# EUROPÄISCHE PATENTSCHRIFT

(11) **EP 0 966 544 B1**
(45) Veröffentlichungstag und Bekanntmachung des Hinweises auf die Patenterteilung: **23.03.2005**
(21) Anmeldenummer: 98922613.9
(22) Anmeldetag: 29.01.1998
(51) Int. Cl.: C12Q 1/68, C12Q 1/00

(54) **TEST-KIT FÜR TUBERKULOSE-DIAGNOSE ETC.**
TEST KIT FOR TUBERCULOSIS DIAGNOSIS OR THE LIKE
NECESSAIRE D'ESSAI POUR LE DIAGNOSTIC DE LA TUBERCULOSE OU ANALOGUE

(30) Priorität: 29.01.1997 EP 97101338
(43) Veröffentlichungstag der Anmeldung: 29.12.1999
(73) Patentinhaber: Lionex GmbH, 38124 Braunschweig (DE)
(72) Erfinder: SINGH, Mahavir, D-38124 Braunschweig (DE); HUTTER, Bernd, D-38124 Braunschweig (DE); KOLK, Arend, D-38124 Braunschweig (DE)
(74) Vertreter: Gramm, Werner, Prof. Dipl.-Ing.
(86) Internationale Anmeldenummer: PCT/EP1998/000483
(87) Internationale Veröffentlichungsnummer: WO 1998/036089

(56) Entgegenhaltungen:
- WO-A-98/32862
- MEDLINE, no. 85008310, Washington DC USA; siehe Zusammenfassung XP002079688 & K.B. KANNAN ET AL.: "Alanine dehydrgenase in mycobacteria: a preliminary report." INDIAN JOURNAL OF LEPROSY, Bd. 56, Nr. 1, 1984, Seiten 98-101, India
- INFECTION AND IMMUNITY, Bd. 60, Nr. 6, 1992, Seiten 2317-2323,

## Beschreibung

Durch Andersen et al. (1992) sind isolierte lambdagt11-Klone bekannt, die die komplette AlaDH kodierende DNA von M. tuberculosis oder Teile davon enthalten. Das isolierte mykobakterielle AlaDH-Insert aus lambdaAA67 wurde dabei als Hybridsierungssonde verwendet.

### 1 Aufgabenstellung und Erfindung

Das in dieser Arbeit behandelte 40 kD-Antigen stellt in vielerlei Hinsicht ein interessantes Objekt für eingehende Studien dar.

Das Antigen war bereits in einen Expressionsvektor für *Escherichia coli* kloniert worden (Konrad & Singh, unveröffentlicht). Es sollte deshalb die Expression und die Aufreinigung des rekombinanten Proteins optimiert werden. Mit einer homogenen Proteinfraktion sollten dann die entscheidenden biochemischen Parameter des Enzym bestimmt werden. Aus solchen Daten lassen sich erfahrungsgemäß Rückschlüsse auf die physiologische Funktion eines Enzyms ziehen. Es stellte sich hierbei die Frage, ob die hypothetische Aufgabe des Enzyms bei der Zellwandbiosynthese unterstrichen oder widerlegt werden kann. Im Falle einer Widerlegung sollten andere mögliche Funktionen eruiert werden.

Zudem können sich aus der Biochemie Ansatzpunkte für eine gezielte Beeinflußung des Enzyms in vivo ergeben. In diesem Zusammenhang ist erneut die physiologische Funktion der Schlüsselpunkt aller Anstrengungen.. Wenn das Antigen für das Bakterium eine essentielle Rolle spielen sollte, dann könnten sich durch gezielte Versuche, das Gen oder das Protein auszuschalten, Möglichkeiten ergeben, den Tuberkulose-Erreger an einem definierten Punkt am Wachstum zu hindern. Das Protein wäre dann ideales drug target. Sollte das 40 kD-Antigen zudem, wie postuliert (Delforge *et al.,* 1993), einen Virulenzfaktor darstellen, dann könnte durch solche Unternehmungen Einfluß auf die natürliche Virulenz des Bakteriums genommen werden. Durch verschiedene Ansätze sollte deshalb auch dieser Punkt überprüft werden.

Die Möglichkeit, mittels des mAb HBT-10 die Stämme *M.tuberculosis* und *M.bovis* BCG zu diskriminieren, ermöglicht es Verfahren zu entwickeln, die eine Infektion von einer Impfung unterscheiden können. Dies ist mit den gebräuchlichen Screeningverfahren, dem PPD- oder dem Mantoux-Test, nicht möglich (Bass Jr. *et al*., 1990; Huebner *et al*., 1993). Durch die Analyse der Verbreitung des Gens bzw. des Genprodukts sollte die Grundlage dafür geschaffen werden, eine rationelle Verfahrensentwicklung für einen solchen Test zu ermöglichen. Zudem sollte untersucht werden, ob das Vorhandensein eines funktionellen Enzyms mit irgendwelchen anderen Parametern korreliert. Insbesondere auf taxonomische und virulente Zusammenhänge wurde hierbei Wert gelegt. Auch bestimmte natürliche Lebensweisen oder der Eintritt in bestimmte Wachstumsphasen könnten mit der Alanin Dehydrogenase in Zusammenhang stehen. Diesen Fragen sollte auf den Grund gegangen werden.

Die Erfindung betrifft nun einen enzymatischen Test-Kit zur Diagnose von Tuberkulose und anderen mycobakteriellen Infektionen in Menschen und Tieren durch Bestimmung der Aktivität von Alanindehydrogenase (E.C. 1.4.1.1) umfassend L-Alanin, Nicotinamidademin-dinucleotid (oxidierte Form; NAD⁺), Phenazinmethosulfat (PMS) und Nitroblue-tetrazoliumchlorid (NBT).

Ferner betrifft die Erfindung ein Verfahren zur Diagnose von Tuberkulose und anderen mycobakteriellen Infektionen von Menschen und Tieren, dadurch ***gekennzeichnet,*** daß man mit einem enzymatischen Test-Kit gemäß Anspruch 1 die Aktivität von Alanindehydrogenase (E.C. 1.4.1.1.) mißt.

Das erfindungsgemäße Verfahren kann dadurch ***gekennzeichnet*** sein, daß man
(i) mögliche Tuberkulose-Erreger, wie *M. tuberculosis* isoliert,
(ii) einen Zellrohextrakt herstellt,
(iii) den Extrakt in Lösung inkubiert und
(iv) die Absorption mißt.

Ferner kann das erfindungsgemäße Verfahren dadurch ***gekennzeichnet*** sein, daß man klinische Proben, wie Köperflüssigkeiten, direkt einer Tuberkulosediagnose unterwirft und die Alanindehydrogenase-Aktivität mißt.

Ferner kann das erfindungsgemäße Verfahren dadurch ***gekennzeichnet*** sein, daß man Zellen, Stämme und/oder Spezies von Krankheitserregern (Mycobakterien) von nicht-virulenten Zellen und Stämmen differenziert.

Ferner kann das erfindungsgemäße Verfahren dadurch ***gekennzeichnet*** sein, daß man Zellen, Stämme und/oder Spezies von Krankheitserregern des *M*. tuberculosis-Komplexes identifiziert und differenziert.

Ferner kann das erfindungsgemäße Verfahren dadurch ***gekennzeichnet*** sein, daß man das Verfahren in Gegenwart von Tuberkulose und anderen mycobakterielle Infektionen von Menschen und Tieren inhibierenden Substanzen durchführt und diese inhibierenden Substanzen gegebenenfalls gewinnt.

Ferner kann das erfindungsgemäße Verfahren dadurch ***gekennzeichnet*** sein, daß man es
(i) zur Epidemiebekämpfung und/oder
(ii) nach Impfungen (vaccination follow-up) bei Menschen und Tieren durchführt.

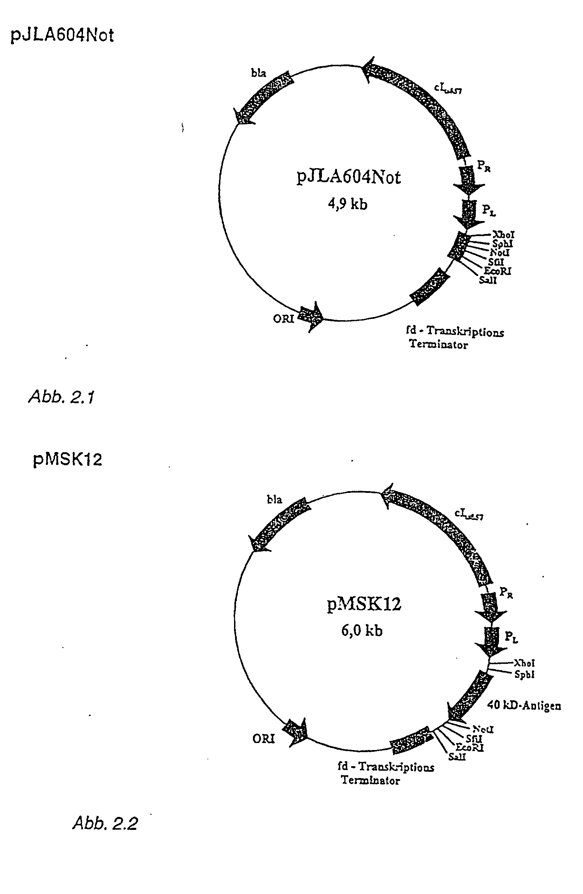

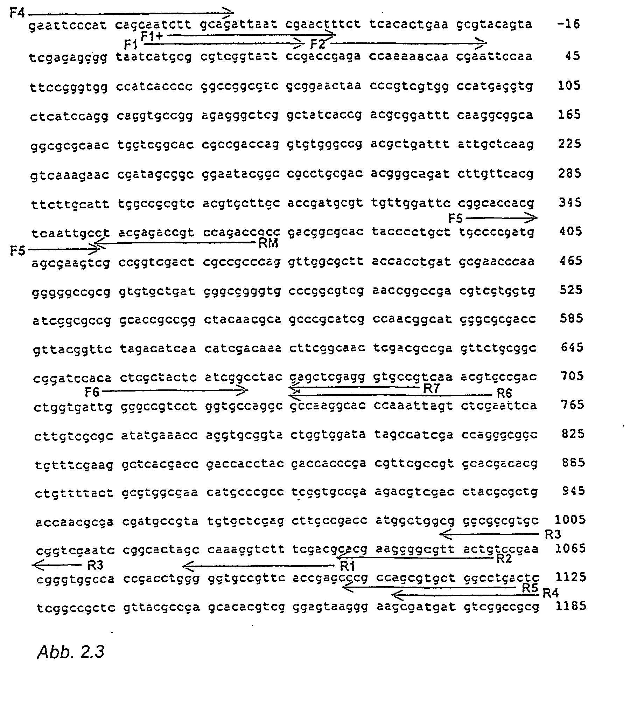

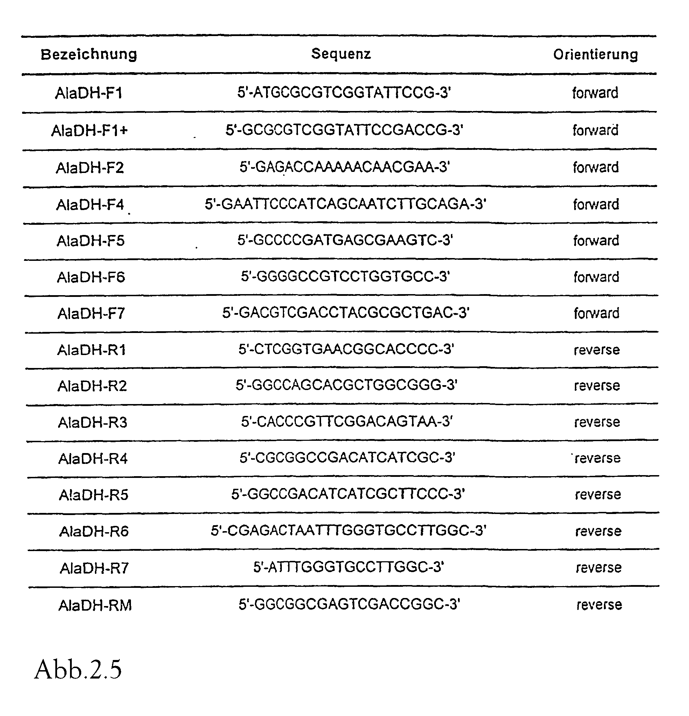

Ferner betrifft die Erfindung eine DNA-Sequenz des Alanindehydrogenase-Gens von *M. tuberculosis*, ausgewählt aus der folgenden Gruppe (Fig. 2.5):

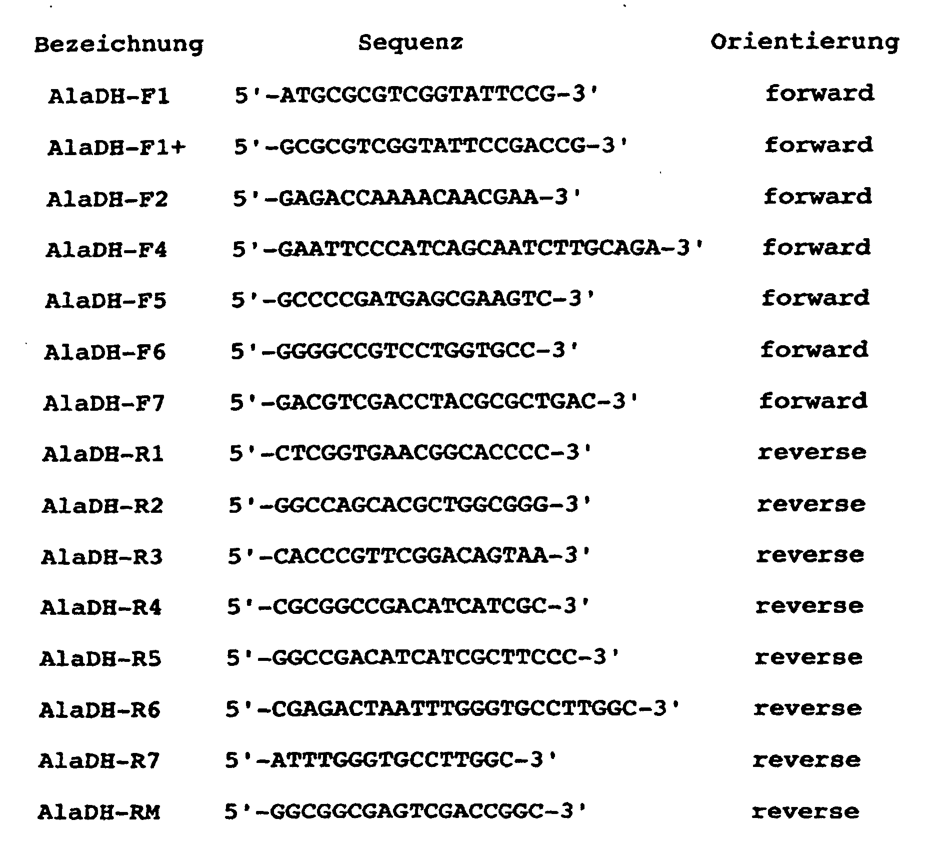

sowie Teilsequenzen davon zur Diagnose von Tuberkulose und anderen mykobakteriellen Infektionen bei Menschen und Tieren.

Die erfindungsgemäße Verwendung einer DNA-Sequenz kann zur Diagnose von Tuberkulose und anderen mycobakteriellen Infektionen bei Menschen und Tieren vorgesehen sein.

Ferner betrifft die Erfindung ein Verfahren, das dadurch ***gekennzeichnet*** ist, daß man eine erfindungsgemäße DNA-Sequenz
(i) zum Hybridisieren,
(ii) zur Bestätigung von Kulturen (culture confirmation) isolierter Stämme und/oder
(iii) für chromosomales Fingerprinting einsetzt und Zellen, Stämme und/oder Arten von Mycobakterien ermittelt und differenziert und/oder für die Diagnose von mycobakteriellen Infektionen einsetzt.

Das erfindungsgemäße Verfahren kann dadurch ***gekennzeichnet*** sein, daß man zellen, Stämme und/oder Spezies von virulenten Mycobakterien von nicht-virulenten Zellen, Stämmen und/oder Spezies differenziert.

Ferner kann das erfindungsgemäße Verfahren dadurch ***gekennzeichnet*** sein, daß man Zellen, Stämmen und/oder Spezies des *M.* tuberculosis-Komplexes und anderer Mycobakterien
(i) isoliert,
(ii) rohe oder gereinigte genomische DNA oder RNA gewinnt,
(iii) ein Fragment identifiziert, das mit der Sequenz des Alanindehydrogenase-Gens von M. *tuberculosis* (Fig. 2.3) identisch oder praktisch identisch ist, vorzugsweise durch Amplifizieren unter Verwendung einer erfindungsgemäßen DNA-Sequenz als Primersequenz, wonach man mit einem Restriktionsenzym verdaut, insbesondere BglII, und eine Gelelektrophorese der verdauten amplifizierten DNA durchführt und/oder die DNA-Sequenz der amplifizierten DNA bestimmt.

Ferner kann das erfindungsgemäße Verfahren dadurch ***gekennzeichnet*** sein, daß man eine klinische Probe unmittelbar einsetzt und auf Tuberkulose bei Menschen und Tieren diagnostiziert.

Ferner kann das erfindungsgemäße Verfahren dadurch ***gekennzeichnet*** sein, daß man das Verfahren in Gegenwart von Tuberkulose oder mycobakterielle Infektionen von Menschen und Tieren inhibierenden Substanzen durchführt und ermittelte inhibierende Substanzen gewinnt oder herstellt.

Ferner kann das erfindungsgemäße Verfahren dadurch ***gekennzeichnet*** sein, daß man es
(i) bei antimycobakterieller Chemotherapie,
(ii) bei der Epidemiebekämpfung und/oder
(iii) nach Impfungen (vaccination follow-up) bei Menschen und Tieren anwendet.

### 2 Materialien und Methoden

### 2.1 Lebendes Material

### 2.1.1 Bakterien

### 2.1.1.1 E. coli Stämme

Der Stamm *Escherichia coli* wurde verwendet um die Expression des rekombinanten 40 kD-Antigens zu optimieren **(Tab. 2.1).** Zudem wurden in ihm bereits klonierte mykobakterielle Antigene überproduziert **(Tab. 2.2).**

**Tab. 2.1:**

| *Benutzte Expressionsstämme und deren relevante Eigenschaften* | | |
|---|---|---|
| Stamm | Genotyp und relevanter Phänotyp | Herkunft / Referenz |
| *E.coli* CAG 629 | *lac*(am) *pho*(am) *trp*(am) *sup*C^{ts} *rps*L *mal*(am) *lon* *htp*R165-Tn10(Tet^{R}) | C.Gross |
| *E.coli* DH5α | *supE44* Δ*lac*U169(φ80 *lac*Z ΔM15) *hsd*R17 *rec*A1 *end*A1 *gyr*A96 *thi-*1 *rel*A1 | Hanahan(1983) |
| *E.coli* TG2 | supE *hsd*Δ5 *thi*Δ(*lac-pro*AB) *Δ*(*srl-rec*A)306::Tn10(Tet^{R}) F'(*tra*D36 *pro*A⁺ *lac*I^{q} *lac*ZM15) | Sambrook *et al*. (1989) |
| *E.coli* SURE | *hsd*R *mcr*A *mcr*B *mvr end*A supE44 *thi*-1 *λ- gyrA96* *rel*A1 *lac rec*B recJ *sbc*C *umu*C *uvr*C (F'*pro*AB *lac*I^{q}Z ΔM15 Tn10(Tet^{R})) | Stratagene |
| *E.coli* BL 321 | *rnc*105 *nad*B⁺ *pur*I⁺ | Studier (1975) |
| *E.coli* N 4830 | *su*^{o} *his ilv gal*KΔ8 *Δchl*D*-pgl* (λ *Δ*Bam N⁺ clₜₛ₈₅₇ ΔHI) | Gottesman *et al*. (1980) |
| *E.coli* 538 | Genotyp unbekannt | Bayer AG |

**Tab. 2.2:**

| *Produzenten mykobakterieller Antigene und deren Charakteristika* | | | | |
|---|---|---|---|---|
| Es ist angegeben, welches Antigen von den jeweiligen Stämmen produziert wird. Die beiden letzten Spalten geben die Anzuchtbedingungen wieder. | | | | |

| Stamm | Herkunft / Referenz(en) | Produkt | Antibiotika | Induktion |
|---|---|---|---|---|
| *E.coli* BL21 (pKAM1301) | J. van Embden | GST-36 kD-Antigen, *M.leprae* | Ap | IPTG |
| *E.coli* BL21/plys5 (pKAM3601) | J. van Embden | 70 kD-Antigen, *M.leprae* | Ap + Cm | IPTG |
| E.coli CAG629 (pMS9-2) | Singh *et al.* (1992) | 38 kD-Antigen, *M.tuberculosis* | Ap | Hitze |
| *E.coli* CAG629 (pMS14-1) | Cherayil & Young (1988) Dale & Patki (1990) Singh *et al.* (unveröff.) | 28 kD-Antigen, *M.leprae* | Ap | Hitze |
| *E.coli* M15 (pHISK16 + pREP4) | Verbon *et al.* (1992) Vordermeier *et al.* (1993) | 16 kD-Antigen, *M.tuberculosis* | Ap | IPTG |
| *E.coli* M1697 | V. Mehra | His-30 kD-Antigen, *M.tuberculosis* | Ap + Km | IPTG |
| *E.coli* M1698 | V. Mehra | His-30 kD-Antigen, *M.leprae* | Ap + Km | IPTG |
| *E.coli* POP (pKAM2101) | J. van Embden | 70 kD-Antigen, *M.tuberculosis* | Ap | Hitze |
| *E.coli* POP (pRIB1300) | Thole *et al.* (1987) van Eden *et al.* (1988) | 65 kD-Antigen, *M.bovis* BCG | Ap | Hitze |
| *E.coli* POP (pZW1003) | Mehra *et al.* (1986) van der Zee *et al.* (unveröff.) | 65 kD-Antigen, *M.leprae* | Ap | Hitze |
| *E.coli* TB1 (pKAM1101) | di Guan *et al.* (1987) Maina *et al*. (1988) Thole *et al.* (1990) | MBP-36 kD-Antigen, *M.leprae* | Ap | Hitze |
| *E.coli* TB1 (pKAM4101) | J. van Embden | MBP-2nd 65 kD-Antigen, *M.leprae* | Ap | Hitze |
| *E.coli* T821-8/2 | Khanolar-Young *et al*. (1992) Mehra *et al*. (1992) | MBP-10 kD-Antigen, *M.tuberculosis* | Ap | IPTG |
| *E.coli* TG2 - 50/55 Sal large | C.Espitia; M.Singh | 50155 kD, large frag., *M.tuberculosis* | Ap | IPTG |

### 2.1.1.2 Mykobakterielle Stämme

**Tab. 2.3:**

| *Benutzte Mykobakterien und deren Herkunft* | | |
|---|---|---|
| Stamm | Abkürzung | Genaue Bezeichnung, Herkunft |
| *M.africanum* 1 | Afr1 | *M.africanum* nr.5544, RIV |
| *M.asiaticum* 1 | Asi1 | *M.asiaticum* 3250, Portaals |
| *M.avium* 1 | Avi1 | *M.avium* Myc 3875, Serotype 2, RIV |
| *M.bovis 3* | Bov3 | *M.bovis* nr.8316, RIV |
| *M.bovis* BCG 2 | BCG2 | *M.bovis* Copenhagen, Serumsinstitut Copenhagen |
| *M.bovis* BCG 4 | BCG4 | *M.bovis* BCG P₃, RIV |
| *M.chelonae 7* | Che7 | *M.chelonei* 1490, P.Dirven |
| *M.flavescens 1* | Fla1 | *M.flavescens* ATCC 14474, RIV |
| *M.fortuitum* 11 | For11 | *M.forfuitum* ATCC 6841, RIV |
| *M.gastri* 1 | Gas1 | *M.gastri* ATCC 25220, RIV |
| *M.gordonae 3* | Gor3 | *M.gordonae* 8960, Portaals |
| *M.intracellulare* 1 | Int1 | *M.intracellulare* 6997, ATCC 15985, Portaals |
| *M.intracellulare 5* | Int5 | *M.intracellulare* IWG MT3, RIV |
| *M.kansasii* 1 | Kan1 | *M.kansasii* Myc 1012, RIV |
| *M.lufu* 1 | Luf1 | *M.lufu* 219, RIV |
| *M.marinum* 3 | Mar3 | *M.marinum* L66, Portaals |
| *M.microti 1* | Mic1 | *M.microti* nr.1278, Portaals |
| *M.nonchromogenium 1* | Non1 | *M.nonchromogenium* ATCC 25145, RIV |
| *M.parafortuitum 1* | Paf1 | *M.parafortuitum* nr.6999, Portaals |
| *M.peregrinum 1* | Per1 | *M.peregrinum*, Patient Bakker, TB6849, Antonie Ziekenhuis |
| *M.phlei* 1 | Phl1 | *M.phlei* 258 (Ph), Portaals |
| *M.phlei* 4 | Phl4 | *M.phlei* Weybridge R82, Tony Eger |
| *M.scrofulaceum 1* | Scr1 | *M.scrofulaceum* Myc 3442, RIV |
| *M.scrofulaceum* 8 | Scr8 | *M.scrofulaceum* Myc 6672, RIV |
| *M.simiae* 1 | Sim1 | *M.simiae* 784, Tony Eger |
| *M.smegmatis* 1 | Sme1 | *M.smegmatis* ATCC 14460, RIV |
| *M.smegmatis 3* | Sme3 | *M.smegmatis* 8070, Portaals |
| *M.terrae* 2 | Ter2 | *M.terrae,* RIV |
| *M.thermoresistibile 1* | The1 | *M.thermoresistibile* nr,7001, Portaals |
| *M.triviale 1* | Tri1 | *M.triviale* 8067, Portaals |
| *M.tuberculosis* H37Rᵥ | H37Rᵥ | *M.tuberculosis* H37Rᵥ, RIV |
| *M.tuberculosis* H37Rₐ | H37Rₐ | *M.tuberculosis* H37Rₐ, nr.19629, RIV |
| *M.tuberculosis* 1 | Tub1 | *M.tuberculosis* 4514, RIV |
| *M.tuberculosis* 49 | Tub49 | *M.tuberculosis* C₃, Sang-Hae Cho, Südkorea |
| *M.tuberculosis* 60 | Tub60 | *M.tuberculosis* S₂, Sang-Hae Cho, Südkorea |
| *M.tuberculosis* 118 | Tub118 | *M.tuberculosis* Myc 16293, Hannoufi |
| *M.tuberculosis* 130 | Tub130 | *M*.*tuberculosis*,patient yy, Strichcode 3.1265, Dr.Bijlmer, Den Haag |
| *M.tuberculosis* 132 | Tub132 | *M.tuberculosis* Myc 16770, RIV |
| *M.tuberculosis* 145 | Tub145 | *M.tuberculosis* 416138N, Patient N.Wielaart, Reg.Nr. 7.796.267, WKZ, Utrecht |
| *M.tuberculosis* 146 | Tub146 | *M.tuberculosis*, Abdi Hussein |
| *M.tuberculosis* 163 | Tub163 | *M.tuberculosis* 925, Patientenisolat Nr. 32, INH>1, Str^{R}, Rit^{S}, Eth^{S} |
| *M. ulcerus 1* | Ulc1 | *M.ulcerus* 932, Portaals |
| *M.vaccae* 3 | Vac3 | *M.vaccae* ATCC 25950, RIV |
| *M.xenopi 7* | Xen7 | *M.xenopicode* 132, Patient Alois Necas, H.Kristanpul, Prag |

### 2.1.1.3 Andere Bakterienstämme

**Tab. 2.4:**

| *Weitere benutzte Bakterienstämme* | |
|---|---|
| Stamm | Herkunft |
| *Listeila monocytogenes* EGB | Andreas Lignau |
| *Listeria innocua* | Andreas Lignau |
| *Nocardia asteroides* 702774 | Juul Bruins |
| *Rodococcus equi* nr.10P388 | VMDC, Utrecht |

### 2.1.2 Zellkultur

- Benutzt wurde die Mäuse Makrophagen Zellinie J774. Diese Zelllinie war ursprünglich aus einem Tumor einer weiblichen BALB/c Maus etabliert worden (Ralph & Nakoinz, 1975). J774 wird für Phagozytose-Assays, zur Produktion von IL-1 und für vielfältige biochemische Untersuchungen benutzt. Sie besitzt Rezeptoren für Immunglobuline und Komplement. Desweiteren produziert J774 Lysozym in großen Mengen und sekretiert IL-1 konstitutiv (Ralph & Nakoinz, 1976; Snyderman *et al.,* 1977). Die Aufnahme von Bakterien erfolgt durch Phagozytose. Direkte Zytolyse von Fremdorganismen ist relativ selten.

### 2.2 Nukleinsäuren

### 2.2.1 Plasmide

Das Plasmid pJLA604Not und seine relevanten funktionellen Abschnitte

Dieses 4,9 kb große Plasmid, ein Derivat von pJLA 604 (Schauder *et al.,1987),* wurde als Expressionsvektor verwendet **(Abb. 2.1).** Das Plasmid pJLA6O4Not (Konrad & Singh, unveröffentlicht) unterscheidet sich von pJLA604 dadurch, daß die *NdeI*-Schnittstelle entfernt, und statt dessen eine NotI-Schnittstelle eingebaut wurde. Das Leseraster der Translation beginnt mit dem ATG-Codon der SphI-Schnittstelle. Die Transkription startet an den Lambda-Promotoren P_{R} und P_{L}, wird jedoch bei Temperaturen von 28-30°C durch das cI_{tS857}-Genprodukt effektiv reprimiert. Induktion wird durch Erhöhung der Temperatur auf 42°C erreicht. Bei dieser Temperatur wird der temperatursensible Lambda-Repressor inaktiv und kann die Transkription nicht mehr reprimieren. Die Transkription endet am fd-Terminator. Zudem besitzt der Vektor die *atpE* Translations-Initiationsregion (TIR) von *E.coli*. Dieser Abschnitt ist sehr nützlich für die Initiativen der Translation, da er nur wenig störende Sekundärstrukturen besitzt und dadurch eine hohe Expressionsrate gewährleistet (McCarthy et al., 1986). Als Selektionsmarker verfügt das Plasmid über das ß-Lactamase-Gen, das für eine Ampicillinresistenz codiert.

Als negatives Kontrollplasmid wurde auch pJLA6O3 verwendet, das bis auf wenige Basen in der Klonierungsstelle mit pJLA6O4 identisch ist.

Das Plasmid pMSKS12 und seine relevanten funktionellen Abschnitte

Dies ist ein Derivat des Plasmids pJLA6O4Not, bei dem zwischen die *SphI-* und die NotI-Schnittstelle das 4OkD-Antigen von *Mycobacterium tuberculosis* kloniert wurde **(Abb. 2.2;** Konrad & Singh, unveröffentlicht).

### 2.2.2 Oligonukleotide

Sämtliche Oligonukleotide **(Tab. 2.5)** wurden von Frau Astrid Hans (GBF, Braunschweig) an einem 394 DNA/RNA Synthesizer (Applied Biosystems) hergestellt. Gereinigt wurden die Oligonukleotide mit einer Oligonudeotide Purification Cartridge (Applied Biosystems).

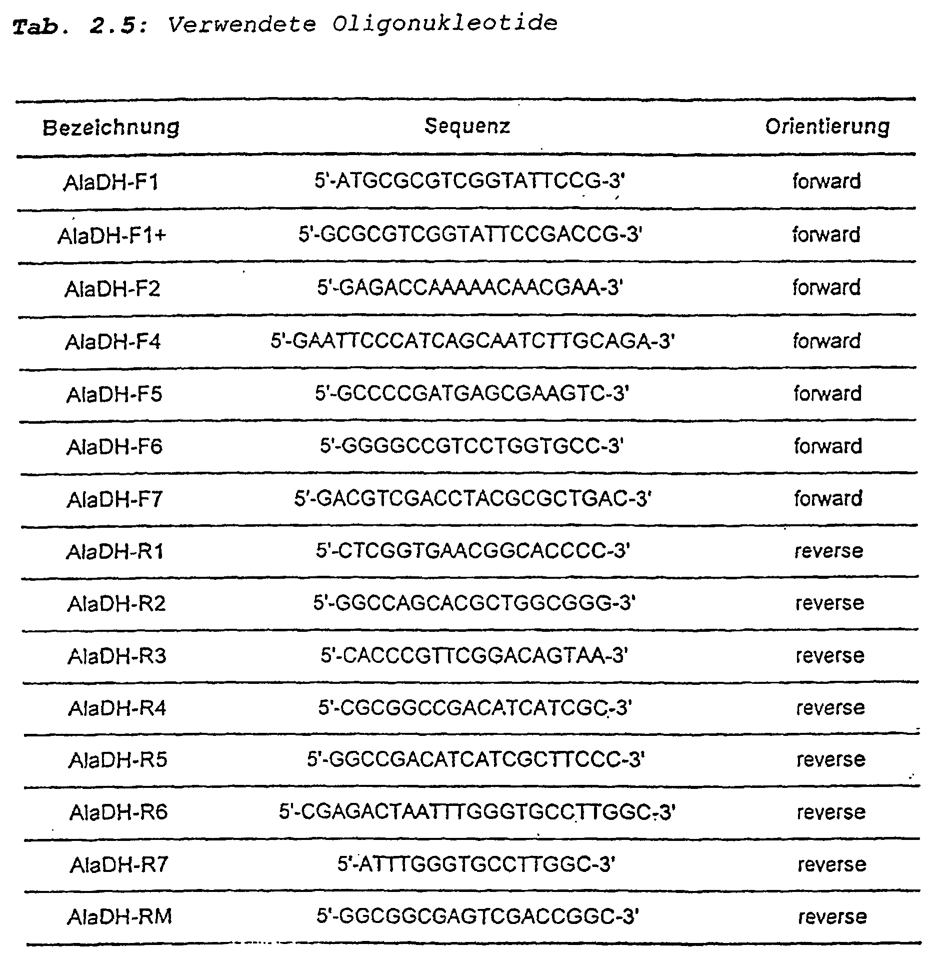

Die Lokalisation der Oligos auf dem *AlaDH-Gen* ist in **Abb. 2.3.** *(Die verwendeten Oligos und ihre Lage auf dem AlaDH-Gen) schematisiert*

### 2.3 Rezepturen

Alle unter diesem Punkt beschriebenen Lösungen wurden weitestgehend nach Sambrook *et al.* (1989) hergestellt.

### 2.3.1 Nährmedien

### LB

10 g Bacto-Trypton (Difco), 5 g Bacto-Hefeextrakt (Difco), 10 g NaCl ad 1000 ml H₂0, pH 7,0, autoklavieren

### IB

12 g Bacto-Trypton (Difco), 24 g Bacto-Hefeextrakt (Difco), 4 ml Glycerin (87 %), 2,31 g KH₂P0₄, 12,54 g K₂HPO₄ ad 1000 ml H₂0, die Phosphatlösungen werden getrennt von den anderen Komponenten autoklaviert und hinterher zugemischt

### SOC

2 % Bacto-Trypton (Difco), 0,5 % Bacto-Hefeextrakt (Difco), 10 mM NaCl, 2,5 mM KCl, 10 mM MgCl₂, 10 mM MgSO₄, 20 mM Glucose ad 1000 ml H₂0, pH 7,0, die Glucose wird getrennt von den anderen Komponen- ten autoklaviert und hinterher zugegeben

### LÖWENSTEIN

Benutzt wurden gebrauchsfertige Coletsos Ossein Schrägagarröhrchen (Sanofi Diagnostics Pasteur).

### FESTMEDIEN:

Zur Herstellung von Platten (90 mm, Greiner) der oben beschriebenen Nährmedien wurde der jeweiligen Rezeptur 1,5% Agar beigemischt.

### ANTIBIOTIKA

Antibiotika wurden den Flüssigmedien kurz vor Gebrauch aus Stammlösungen zugegeben. Bei Herstellung von Festmedien wurde mit der Zugabe gewartet, bis die Lösung nach dem Autoklavieren handwarm war. Benutzt wurden die in Tab. 2.6 aufgeführten Antibiotika.

**Tab. 2.6:**

| *Benutzte Antibiotika und eingesetzte Konzentrationen* | | |
|---|---|---|
| Antibiotika | Endkonzentration | gelöst in |
| Ampicillin | 100 µg/ml | Wasser |
| Chloramphenicol | 20 µg/ml | Ethanol |
| Gentamicin | 100 µg/ml | gebrauchsfertig (Sigma) |
| Kanamycin | 30 µg/ml | Wasser |

### 2.3.2 Pufferlösungen

- L-PUFFER:: 50 mM Tris base, 10 mM EDTA, pH 6,8, autoklavieren
- TE:: 10 mM Tris base, 1 mM EDTA, pH 7,4, autoklavieren
- TAE:: 40 mM Tris-Acetat, 1 mM EDTA, pH 8,0, autoklavieren
- TBE:: 89 mM Tris base, 89 mM Borsäure, 2 mM EDTA, pH 8,0,
- TBS:: 50 mM Tris base, 137 mM NaCl, 3 mM KCl, pH 7,4, autoklavieren
- TBS-TWEEN:: TBS + 0,05 % Tween-20
- PBS:: 137 mM NaCl, 3 mM KCl, 8 mM Na₂HPO₄, 2 mM KH₂PO₄, pH 7,0, autoklavieren

### 2.4 Alanin Dehydrogenase Assays

### 2.4.1 Qualitativer Assay

Der qualitative Nachweis der AlaDH beruht auf einer Reihe von Redoxreaktionen, gemäß folgendem Reaktionsschema (Inagaki *et al.,* 1986; Andersen *et al.,* 1992):

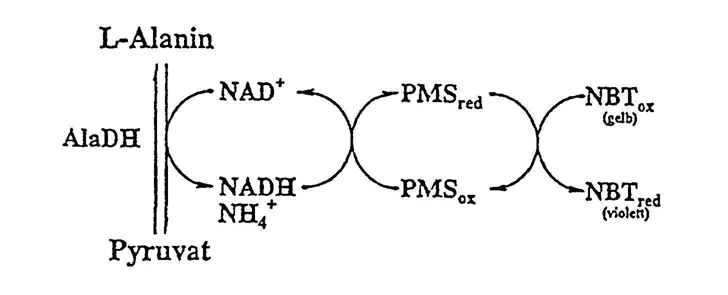

### Prinzip des Alanin Dehydrogenase-Assays

Das violette Endprodukt ist hierbei sehr gut mit dem bloßen Auge zu erkennen. Dieser Assay wurde einerseits zum schnellen Screening von FPLC-Fraktionen und andererseits zur Demonstration von AlaDH-Akti-vität in nativen Proteingelen benutzt.

Basis dieses Assay ist ein Reaktionsmix bestehend aus 1/2 Vol. 0,5 M Glycin-KOH, pH 10,2 und je 1/8 Vol. 0,5 M L-Alanin, 6,25 mM NAD⁺, 2,4 mM NBT und 0,64 mM PMS.

Zur Analyse von Proteinfraktionen wurde der Substratmix 1:1 mit der zu testenden Lösung versetzt. Native Gele wurden nach der Elektrophorese direkt in 10 ml Substratmix inkubiert.

Eine positive Reaktion ist nach spätestens 5 min zu erkennen.

### 2.4.2 Semiquantitativer Assay

Dieser Assay wurde zur Untersuchung von AlaDH-Aktivitäten in Mykobakterien verwendet.

Die Mykobakterien wurden auf Löwenstein-Medium angezogen. Mit der Impföse wurden Bakterien von den Schrägagar-Röhrchen abgenommen, in Wasser resuspendiert und auf eine Trübung äquivalent zu einem McFarland Standard Nr. 5 eingestellt. Zur Trennung von Zellaggregaten wurden die Suspensionen für 10 min im Ultraschallbad behandelt.

Anschließend wurden die Zellen 1:1 mit Reaktionsmix (siehe 2.4.1) versetzt und 10 min bei RT inkubiert. Nach zweiminütiger Zentrifugation bei 20.000 g wurde die Absorption des Überstandes gegen den Blindwert gemessen.

Als Referenzmessung diente ein Ansatz, dem kein L-Alanin zugesetzt war. Eine Absorptionsänderung von einer Einheit pro Minute in diesem Test entspricht etwa einer Absorptionsänderung von drei Einheiten pro Minute beim quantitativen Assay (Messung bei 340 nm, siehe 2.4.3).

### 2.4.3 Quantitativer Assay

Bei diesem Assay wurde direkt die quantitative Änderung des NADH-Gehaltes bei 340 nm gemessen.

Die Standardreaktionsansätze hatten ein Volumen von 1 ml. Die Zusammensetzung ist in **Tab. 2.7** gezeigt. Die Absorption wurde über 10 min hinweg bei 37 °C und 340 nm verfolgt. Der Extinktionskoeffizient ε von NADH beträgt bei 340 nm 6,22 x 10⁶ cm²/ mol.

Die Standardansätze wurden zur Bestimmung der biochemischen Eigenschaften des Enzyms wie jeweils im Text angegeben variiert. Jeder dargestellte Meßpunkt stellt den Mittelwert aus mindestens zwei, in der Regel aber drei, unabhängigen Messungen dar.

Eine AlaDH-Einheit ist als die Enzymmenge definiert, die in einer Minute die Bildung von 1 µmol NADH in der oxidativen Desaminierung katalysiert.

**Tab. 2.7:**

| Zusammensetzung des quantitativen AlaDH-Assays | |
|---|---|
| Die Zusammensetzung des Reaktionsansatzes für die oxidative Desaminierung ist links, die für die reduktive Aminierung rechts gezeigt. | |

| Oxidative Desaminierung | Reduktive Aminierung |
|---|---|
| 125 mM Glycin·KOH, pH 10,2 | 1 M NH₄Cl / NH₄OH, pH 7,4 |
| 100 mM L-Alanin | 20 mM Pyruvat |
| 1,25 mM NAD⁺ | 0,5 mM NADH |

### 3. Die Verbreitung der Alanin Dehydrogenase innerhalb der Mykobakterien

Sowohl auf Gen-, als auch auf Proteinebene, sollte als nächstes untersucht werden, in welchen Mykobakterien eine Alanin Dehydrogenase vorhanden ist. Ausgehend von der Virulenz war hierbei die Fragestellung, ob die AlaDH-Aktivität mit dieser Eigenschaft korreliert.

### 3.1 In vivo AlaDH-Aktivität

Da AlaDH-Aktivität in der Mikrobenwelt eher die Ausnahme als die Regel darstellt, war es interessant zu hinterfragen, ob dieses Enzym innerhalb der Mykobakterien ubiquitär ist, oder ob es auf bestimmte Spezies und Stämme beschränkt ist. Dadurch können dann wiederum Rückschlüsse auf Fragen gezogen werden wie:
Haben AlaDH produzierende Stämme Gemeinsamkeiten in der Lebensweise?
Induziert eine bestimmt Wachstumsweise oder -phase die AlaDH-Produktion?
Wie erfolgt die Regulation der AlaDH?
Können andere Stoffwechselwege die von der AlaDH katalysierte Reaktion ersetzen?
Welchen Phänotyp müssten *AlaDH* Mutanten zeigen?

Es wurden deshalb alle verfügbaren Stämme auf Produktion von AlaDH-Aktivität hin untersucht. Das Repertoir umfasste insgesamt 44 mykobakterielle Stämme, die 29 verschiedene Spezies repräsentieren. Zudem wurden die beiden mit den Mykobakterien eng verwandten Stämme *Nocardia asteroides* und *Rhodococcus equi* getestet.

Um die im Testsystem gemessenen Aktivitäten miteinander vergleichen zu können, wurden alle Bakteriensuspensionen auf eine Dichte eingestellt, die der Trübung eines McFarland Standards Nr. 5 entspricht. Die Stämme befanden sich zum Zeitpunkt der Messung in der späten exponentiellen Phase.

Neben der AlaDH-Messung wurde auch eine Messung durchgeführt, bei der im Reaktionsansatz L-Alanin fehlte. Die Aktivität dieses Ansatzes ist ein Maß für andere parallel ablaufende NAD⁺-reduzierende Prozesse. Die Differenz zwischen diesem Ansatz und dem Standardansatz entspricht der Netto-AlaDH-Aktivität (ΔA₅₉₅-Wert).

Gemäß der gemessen Aktivitäten lassen sich die untersuchten Stämme in drei Gruppen einteilen. Die erste Gruppe ist die der stark-positiven Stämme **(Tab.3.1).** In dieser Gruppe sind die Stämme zusammengefasst, die eine AlaDH-Aktivität von mehr als 0,5 ΔA₅₉₅-Einheiten im benutzen Testsystem aufweisen.

**Tab. 3.1:**

| Stämme mit stark-positiver AlaDH-Aktivität | |
|---|---|
| Die Durchführung dieses Assays ist in 2.4.2 beschrieben. | |

| Stamm | AlaDH-Aktivität [ΔA₅₉₅] |
|---|---|
| *M.marinum 3* | 2,327 |
| *M.chelonae 7* | 1,842 |
| *M.microti* 1 | 0,919 |
| *M.tuberculosis* H37Rᵥ | 0,592 |

Als stark-postiv klassifiziert wurden die beiden für Fische pathogenen Stämme M.chelonae und M.marinum, sowie die beiden ebenfalls pathogenen Stämme *M.microti* und *M.tuberculosis* H37Rᵥ, letzteres ein virulenter Tuberkulose-Referenzstamm.

Die zweite Gruppe, die der mäßig-positiven Stämme, umfasst jene mit einer Aktivität zwischen 0,1 und 0,5 ΔA₅₉₅-Einheiten **(Tab.3.2)**.

**Tab. 3.2:**

| *Stämme mit mäßig-positiver AlaDH-Aktivität* | | | |
|---|---|---|---|
| Die Durchführung dieses Assays ist in 2.4.2 beschrieben. | | | |

| Stamm | AlaDH-Aktivität [ΔA₅₉₅] | Stamm | AlaDH-Aktivität [ΔA₅₉₅] |
|---|---|---|---|
| *M.smegmatis* 3 | 0,375 | *M.tuberculosis* 49 | 0,138 |
| *M.ulcerus* 1 | 0,369 | *M.tuberculosis* 130 | 0,118 |
| *M.africanum* 1 | 0,287 | *M.smegmatis* 1 | 0,116 |
| *M.tuberculosis* 118 | 0,210 | *M.tuberculosis* 132 | 0,111 |
| *M.tuberculosis* 145 | 0,190 | *M.tuberculosis* 146 | 0,111 |
| *M.intracellulare* 1 | 0,155 | *M.tuberculosis* 1 | 0,110 |

In dieser Gruppe finden sich, außer *M.smegmatis,* nur pathogene, klinische Isolate von *M.tuberculosis* und anderen Mykobakterien wieder. Beide getesteten Stämme von *M.smegmatis* zeigen jedoch auch sehr hohe NAD⁺-reduzierende Aktivitäten in Abwesenheit von L-Alanin. Es ist an dieser Stelle noch wichtig zu erwähnen, daß der Stamm *M.smegmatis* 1-2c (ein Derivat von *M.smegmatis* mc²6; Zhang *et al.,* 1991; Garbe *et al.,* 1994; von Dr. Peadar Ó Gaora, St. Mary's Hospital, London), ein Stamm für genetische Arbeiten in Mykobakterien, keine AlaDH-Aktivität zeigt, aber ebenfalls über eine hohe Hintergrundaktivität verfügt.

In der letzten Gruppe schließlich sind alle Stämme aufgeführt, die als für AlaDH-Aktivität negativ befunden wurden, d.h. die eine Aktivität von weniger als 0,1 ΔA₅₉₅-Einheiten aufweisen **(Tab.3.3).**

**Tab. 3.3:**

| *Stämme ohne AlaDH-Aktivität* | | | |
|---|---|---|---|
| Die Durchführung dieses Assays ist in 2.4.2 beschrieben. | | | |

| Stamm | AlaDH-Aktivität [ΔA₅₉₅] | Stamm | AlaDH-Aktivität [ΔA₅₉₅] |
|---|---|---|---|
| *N.asteroides* 1 | 0,048 | *M.bovis* BCG 4 | 0,001 |
| *M.flavescens* 1 | 0,042 | *M.terrae* 2 | 0,001 |
| *M.tuberculosis* H37Rₐ | 0,032 | *M.tuberculosis* 60 | 0 |
| *M.nonchromogenium* 1 | 0,026 | *M.tuberculosis* 163 | 0 |
| *M.fortuitum* 11 | 0,022 | *M.gastri 1* | 0 |
| *M.asiaticum* 1 | 0,021 | *M.gordonae* 3 | 0 |
| *M.bovis* BCG 2 | 0,013 | *M.kansasii 1* | 0 |
| *M.lufu* 1 | 0,013 | *M.parafortuitum 1* | 0 |
| *R.equi* 1 | 0,011 | *M.perigrinum 1* | 0 |
| *M.bovis* 3 | 0,010 | *M.phlei* 1 | 0 |
| *M.scrofulaceum* 1 | 0,009 | *M.phlei 4* | 0 |
| *M.intracellulare* 5 | 0,007 | *M.scrofulaceum 8* | 0 |
| *M.thermosresistibile 1* | 0,006 | *M.simiae 1* | 0 |
| *M.avium 1* | 0,002 | *M.vaccae* 3 | 0 |
| *M.triviale 1* | 0,002 | *M.xenopi* 7 | 0 |

Diese weitaus größte Gruppe umfaßt vor allem opportunistische und nicht-pathogene Stämme, sowie die beiden mit den Mykobakterien verwandten Stämme *Nocardia asteroides* und Rhodococcus *equi.*

Ausnahme waren zwei klinische Tuberkulose-Isolate, sowie der Erreger der Rinder-Tb, *M.bovis,* aber auch die beiden untersuchten Impfstämme von *M.bovis* BCG.

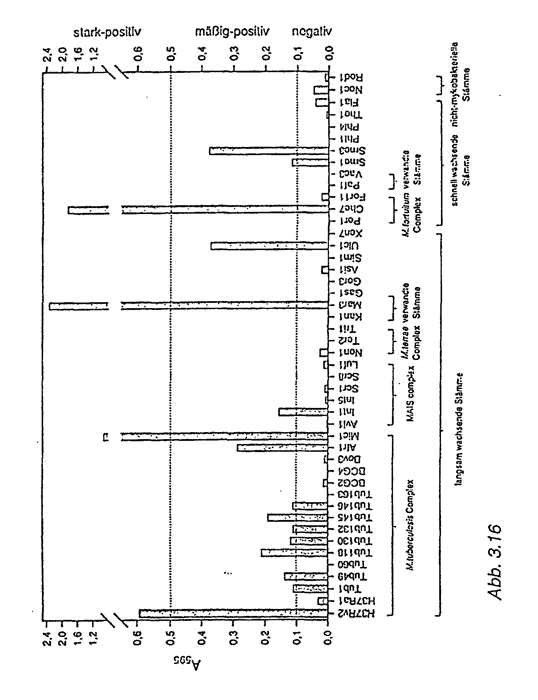

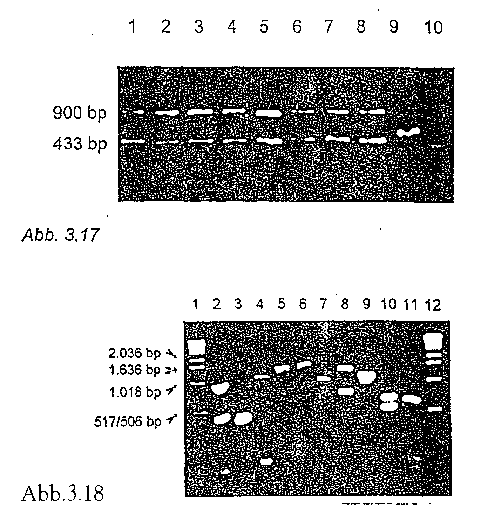

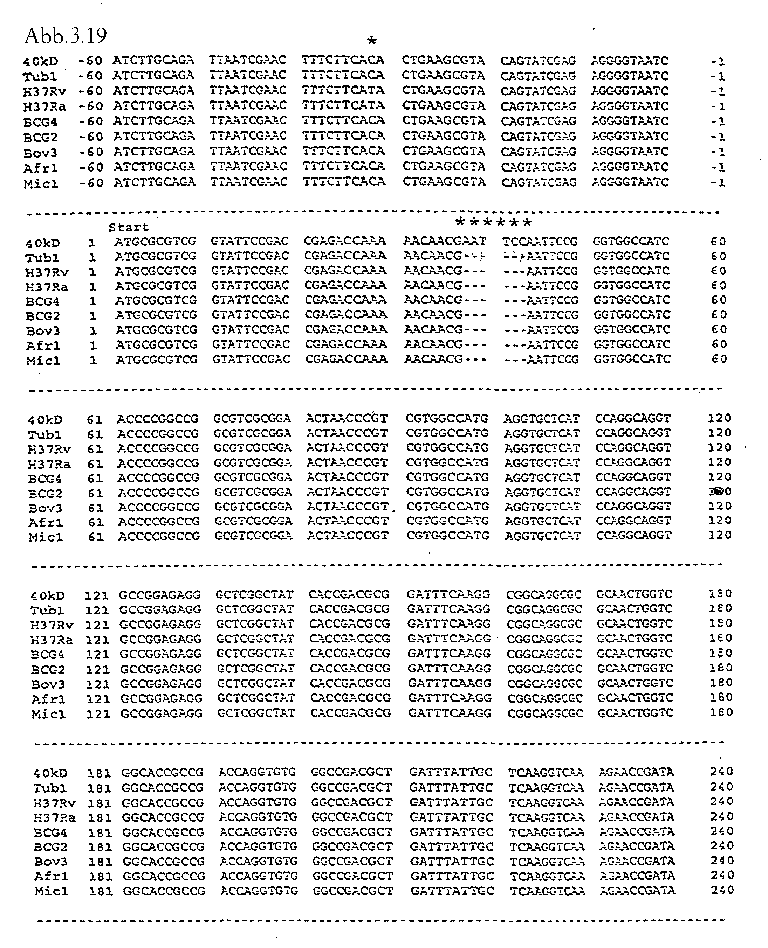

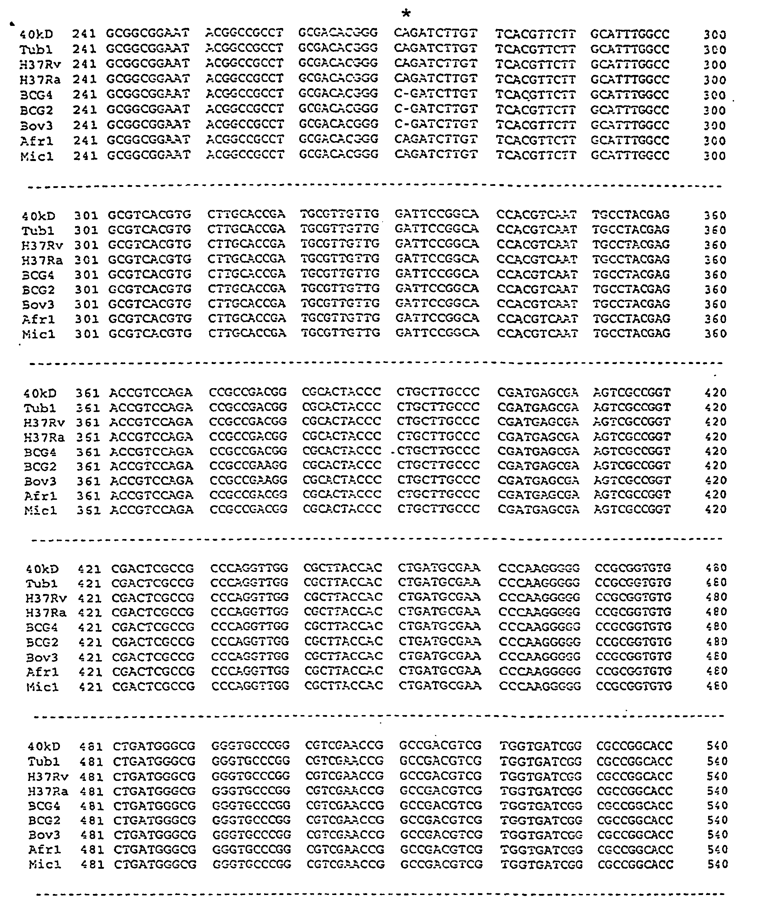

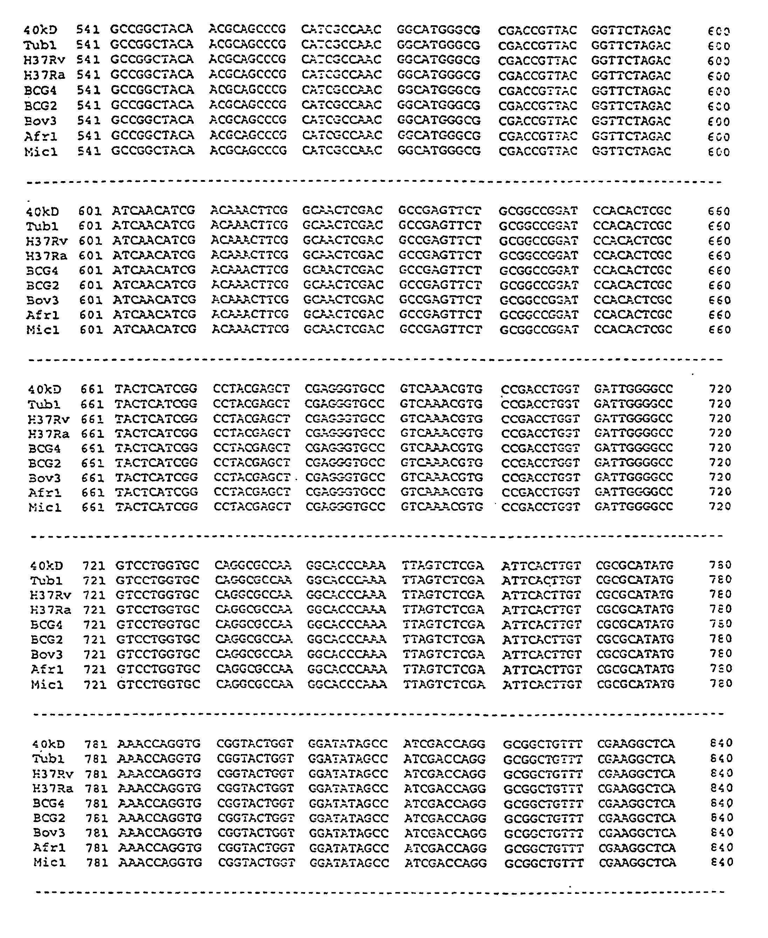

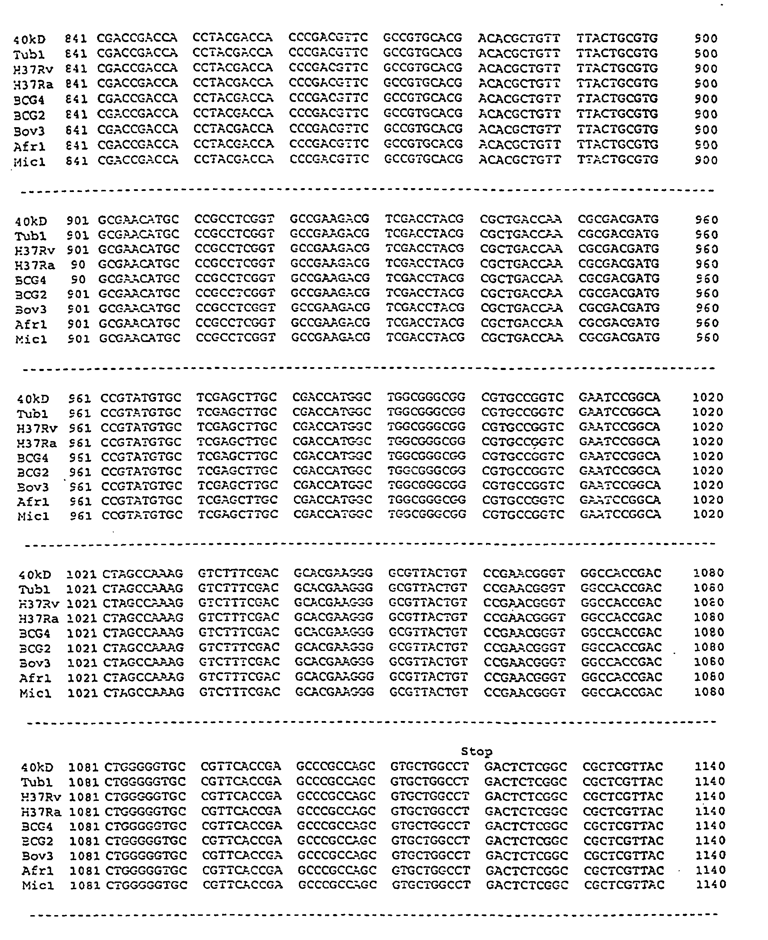

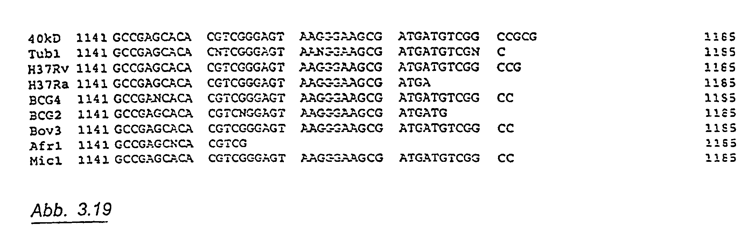

Eine graphische Darstellung der AlaDH-Aktivitäten im Reich der Mykobakterien ist in **Abb. 3.16** nach phylogenetischen Gesichtspunkten geordnet wiedergegeben.

Die genaue Bezeichnung der einzelnen Stämme ist in **Tab. 2.3** wiedergegeben. Die Angaben *schnell wachsend* bzw. *langsam wachsend* dürfen nicht streng genommen werden, sondern stellen vielmehr eine Tendenz innerhalb der gezeigten Gruppen dar.

Zusammenfassend läßt sich die Verbreitung von AlaDH-Aktivität innerhalb der Welt der Mykobakterien so beschreiben:
① Die mit Abstand höchste Aktivität zeigen die beiden für Fische pathogenen Stämme *M.chelonae* und *M.marinum.*
② Innerhalb der Stämme von *M.tuberculosis* ist eine Tendenz vorhanden, nach der mit abnehmender Virulenz auch die AlaDH-Aktivität abnimmt (H37Rᵥ > klinische Isolate > H37Rₐ).
③ Alle als positiv klassifizierten Stämme sind virulent. Einzige Ausnahme ist M.*smegmatis*, das aber anhand seiner hohen Hintergrundaktivität leicht zu unterscheiden ist.
④ Nicht alle virulenten Stämme sind AlaDH positiv.
⑤ *M*. *tuberculosis* läßt sich mittels AlaDH-Aktivität vom Impfstamm *M.bovis* BCG unterscheiden.

### 3.2 Das Gen für die Alanin Dehydrogenase

### 3.2.1 Die ersten PCR-Fragmente

Nachdem nun die AlaDH-Aktivitäten innerhalb der verschiedenen Stämme quantifiziert worden waren, stellte sich als nächstes die Frage, warum einige Stämme das Enzym produzieren, andere aber nicht. Auch das Ausmaß der Expression unterscheidet sich selbst zwischen eng verwandten Arten teilweise deutlich.

Das Fehlen meßbarer Aktivität kann bis zu einem gewissen Grad eine Erklärung darin finden, daß sich nicht alle Stämme in der exakt selben Wachstumsphase befanden, da es sehr schwer ist alle Stämme parallel, im gleichen Stadium befindlich anzuziehen. Aber das Ausbleiben von Aktivität könnte auch einen Grund darin haben, daß sich genetische Änderungen auf die Expression des Gens auswirken. Diese Änderungen könnten im kodierenden oder im regulatorischen Bereich aufgetreten sein.

Um diese Tatsache zu überprüfen wurde versucht das *AlaDH-Gen* aus verschiedenen Stämmen mittels PCR ganz oder teilweise zu amplifizieren. Als Primer dienten hierzu auf der Sequenz von *M.tuber-culosis* H37Rᵥ basierende Oligonukleotide (Andersen et *al.,* 1992; siehe Abschnitt 2.2.2, **(Tab. 2.5).**

Die zum Nachweis der AlaDH verwendeten Primerpaare, die jeweils zu erwartende Länge der Produkte und die jeweils benutzten Annealing-Temperaturen der PCR sind in **Tab. 3.4** zusammengefasst.

**Tab. 3.4:**

| Primerpaare zur Detektion der AlaDH in Mykobakterien | | | | |
|---|---|---|---|---|
| Die Sequenzen der Primer sind in **Tab. 2.5** wiedergegeben. | | | | |

| Bezeichnung | Primer #1 | Primer#2 | Produkt | Temperatur |
|---|---|---|---|---|
| *Annabel* | AlaDH-F1 | AlaDH-RM | 433 bp | 65°C |
| *Beatrice* | AlaDH-F1 | AlaDH-R2 | 1102 bp | 45°C |
| *Claudette* | AlaDH-F1 | AlaDH-R3 | 1120 bp | 55°C |
| *Désirée* | AlaDH-F1 | AlaDH-R6, | 1072 bp | 45°C |
| *Eleonore* | AlaDH-F1+ | AlaDH-R1 | 1099 bp | 55°C |
| *Francoise* | AlaDH-F1+ | AlaDH-R2 | 1117 bp | 50°C |
| *Giselle* | AlaDH-F2 | AlaDH-R7 | 757 bp | 35°C |
| *Helen* | AlaDH-F4 | AlaDH-RM | 1080 bp | 55°C |
| *Isabelle* | AlaDH-F4 | AlaDH-R6 | 1050 bp | 55°C |
| *Jeanette* | AlaDH-F5 | AlaDH-R1 | 507 bp | 45°C |
| *Karen* | AlaDH-F5 | AlaDH-R4 | 834 bp | 45°C |
| *Larissa* | AlaDH-F6 | AlaDH-R4 | 786 bp | 55°C |
| *Melanie* | AlaDH-F6 | AlaDH-R5 | 405 bp | 55°C |

Die ersten Versuche, das Gen für die AlaDH in verschiedenen mykobakteriellen Spezies zu detektieren, erfolgte mit dem Primerpaar *Annabel.* Das hierbei erhaltene Ergebnis war einigermaßen überraschend. Alle Stämme des *M.tuberculosis* Complex zeigten das erwartete Fragment von 433 bp. Darüber hinaus war bei all diesen Stämmen ein zusätzliches Fragment von etwa 900 bp amplifiziert worden **(Abb. 3.17):**
. PCR verschiedener Stämme mit dem Primerpaar Annabel.
. Gefahren wurden bei diesen PCR's jeweils 40 Zyklen mit der Abfolge: *melting* 2 min bei 96 °C, *annealing* 2 min bei 65 °C und *extension* 3 min bei 72 °C. Die MgCl₂-Konzentration betrug 1,5 mM.

| | | | |
|---|---|---|---|
| Bahn 1 | *M.tuberculosis* H37Rᵥ | Bahn 6 | *M.bovis* BCG 4 |
| Bahn 2 | *M.tuberculosis* H37Rₐ | Bahn 7 | *M.africanum* |
| Bahn 3 | *M.tuberculosis 1* | Bahn 8 | *M.microti 1* |
| Bahn 4 | *M.bovis 3* | Bahn 9 | *M.marinum 3* |
| Bahn 5 | *M.bovis* BCG 2 | Bahn 10 | *M.chelonae 7* |

Wie sich herausstellen sollte, war dieses zweite Fragment ebenfalls ein Teil des *AlaDH-Gens,* das durch Anlagerung des Primers AlaDH-RM an einer weiter C-terminal gelegenen Stelle entstanden ist. Durch Erhöhung der Annealing-Temperatur bei der PCR von 65 auf 69 °C konnte dieses zweite Fragment unterdrückt werden (siehe **Abb. 3.18,** Bahn 2 und 3).

Das eigentlich erstaunliche war jedoch das Erscheinen des amplifizierten Fragments in allen Stämmen des *M.tuberculosis* Complex, unabhängig vom Vorhandensein von AlaDH-Aktivität.

Auch bei einigen anderen Stämmen konnte mit dem Primerpaar Annabel eines oder mehrere Fragmente amplifiziert werden. Allerdings waren die amplifizierten Banden meist nicht besonders stark, so daß sie in Anbetracht der 40 PCR-Zyklen als Hintergrund betrachtet werden können. Es handelt sich vermutlich um schwache unspezifische Reaktionen. Jedoch ist auch nicht auszuschliessen, daß aufgrund mangelnder Homologie zwischen den verschiedenen Spezies die PCR-Primer nicht optimal an die Zielsequenz binden konnten.

Die beiden fischpathogenen Stämme mit starker AlaDH-Aktivität, *M.marinum* und *M.chelonae,* zeigten bei der PCR mit dem Primerpaar *Annabel* ein deutlich unterschiedliches Verhalten. Während *M.marinum* ein Produkt von etwa 540 bp lieferte, war bei den gewählten Bedingungen mit dem Primerpaar *Annabel* bei *M.chelonae* kein Fragment zu erhalten **(Abb. 3.17,** Bahn 9 und 10).

### 3.2.2 Das AlaDH-Gen des M.tuberculosis Complex

Da die Präsenz des Gens für die AlaDH nun in allen Stämmen des M.tuberculosis Complex nachgewiesen worden war, stellte sich die Frage, wie man sich die Diskrepanz zu den gemessenen Aktivitäten erklären sollte.

Aus diesem Grunde wurde damit begonnen, größere Fragmente des Gens zu amplifizieren. Aus *M.tuberculosis* H37Rᵥ konnten alle in **Tab. 3.14** aufgeführten Fragmente amplifiziert werden (ein Teil dieser Fragmente ist in **Abb. 3.18** gezeigt). Von den anderen Stämmen des *M.tuberculosis* Complex sind ebenfalls alle PCRReaktionen aus **Tab. 3.15,** die ausprobiert wurden, positiv verlaufen. Es wurde jedoch nicht mit jedem Stamm jede Reaktion nachvollzogen.

### PCR-Produkte des Stammes M. tuberculosis H37Rᵥ

Gefahren wurden bei diesen PCR's jeweils 40 Zyklen wie in **Abb. 3.17** angegeben. Die annealing-Temperaturen sind, mit Ausnahme von Bahn 2 und 3, in **Tab. 3.14** wiedergegeben. Die MgCI₂-Konzentration beim Primerpaar *Annabel* betrug 1,5 mM, die aller anderen Reaktionen 3 mM.

| | | | |
|---|---|---|---|
| Bahn 1 | KBL | Bahn 7 | *Giselle* |
| Bahn 2 | *Annabel, 65 °C* | Bahn 8 | *Helen* |
| Bahn 3 | *Annabel, 69 °C* | Bahn 9 | *Isabelle* |
| Bahn 4 | *Désirée* | Bahn 10 | *Larissa* |
| Bahn 5 | *Eleonore* | Bahn 11 | *Melanie* |
| Bahn 6 | *Francoise* | Bahn 13 | KBL |

Der von allen Stämmen des *M.tuberculosis* Complex amplifizierte Bereich umfasst 1260 bp. Er beinhaltet den kompletten kodierenden Abschnitt für die AlaDH, sowie weitere 75 bp upstream und 63 bp *downstream.* Dieser Bereich wurde von allen Stämmen des *M.tuberculosis* Complex komplett durchsequenziert **(Abb. 3.19).** Lediglich bei den letzten etwa 20 Basen schleichen sich Ungenauigkeiten ein. Der komplette restliche Bereich ist jedoch durch mehrfache Sequenzierungen abgesichert.

Es ist festzustellen, daß sämtliche Sequenzen bis auf drei Stellen komplett mit der publizierten Sequenz der AlaDH des λAA65-Klons (Andersen *et al,* 1992) identisch sind.

Alignment des AlaDH-Gens und den flankierenden Regionen aus verschiedenen Stämmen des *M. tuberculosis Complex*

Die mit "40kD" bezeichnete Zeile gibt die Sequenz von Andersen *et al*. (1992) wieder. Sequenzunterschiede sind jeweils mit einem "*" über der Sequenz gekennzeichnet. Das Start- und das Stopcodon sind ebenfalls über der Sequenz markiert. Bei den fett gedruckten Basen am Ende der Sequenz handelt es sich um Sequenzierungenauigkeiten.

Die erste Stelle, an der sich die Sequenzen unterscheiden, ist Base -32, also *upstream* des Translations-Startsignals. Interessanterweise unterscheiden sich an dieser Stelle die in dieser

Arbeit bestimmten Sequenzen von *M.tuberculosis* H37Rᵥ und H37Rₐ von der Sequenz von Andersen und Mitarbeitern (Andersen *et al.,* 1992). Alle anderen Sequenzen die in dieser Arbeit untersucht wurden, einschließlich die des dritten getesteten Stammes von *M.tuberculosis*, stimmen mit der Sequenz von Andersen überein.

Dies ist insofern verwunderlich, als daß die ursprünglich publizierte Sequenz auf dem Klon einer λgt11-Bank beruht, die aus dem Stamm *M.tuberculosis* H37Rᵥ hergestellt worden war. Es wurde deshalb der Frage nachgegangen, ob eventuell durch die PCR ein Fehler eingeführt worden war. Dies bestätigte sich jedoch nicht. Es könnte jedoch auch möglich sein, daß der in dieser Arbeit benutzte Stamm von *M.tuberculosis* H37Rᵥ einen anderen Ursprung hat als der von Andersen. Ähnliche kleine Varianzen sind auch bei verschiedenen Stämmen unterschiedlichen Ursprungs von *M*.*bovis* BCG bekannt.

An der zweiten Stelle unterscheiden sich alle Stämme des *M.tuberculosis* Complex von der publizierten Sequenz der AlaDH von *M.tuberculosis* H37Rᵥ. Es handelt sich um die Region der Basen 38 bis 49. Innerhalb dieser zwölf Basen wird die Sequenz AATTCCwiederholt, die Basen 44 bis 49 stellen also ein direct repeat der Basen 38 bis 43 dar. In allen acht sequenzierten Stämmen ist dieses Muster jedoch jeweils nur einmal zu finden. Es ist also davon auszugehen, daß sich bei der von Andersen *et al.* (1992) bestimmten Sequenz ein Sequenzierungs- oder Lesefehler eingeschlichen hat. Die Gensequenz und die davon abgeleitete Aminosäuresequenz ändert sich hierdurch folgendermaßen:
Andersen *et al*., 1992: 
Gensequenz        A A C G A A T T C C A A T T C C G G G T G
Proteinsequenz      Asn    Glu    Phe    Gln    Phe    Arg    Val
Diese Arbeit:

Gensequenz      A A C G A A T T C - - - - - - - C G G G T G
Proteinsequenz  Asn   Glu   Phe    -    -    Arg   Val

Es handelt sich also effektiv um den 'Verlust' der beiden Aminosäuren Glutamin und Phenylalanin. Nach dieser Deletion setzt sich die Sequenz wie von Andersen *et al*. (1992) publiziert fort.

Dieser Sachverhalt wurde durch die N-terminale Sequenzierung des Proteins bestätigt. Weder im nativen Protein von *M.tuberculosis* H37Rᵥ, noch im rekombinanten Protein aus *F.coli,* waren die beiden Aminosäuren zu finden.

Bei der dritten unterschiedlichen Stelle handelt es sich um Base 272. An dieser Stelle befindet sich, mit der Ausnahme dreier. Stämme, ein Adeninrest. Bei diesen drei Stämmen, *M.bovis* und zwei Stämme von *M.bovis* BCG, ist diese Base deletiert. Diese Deletion führt zu einer Leserasterverschiebung, die den gesamten folgenden Teil des resultierenden Proteins betrifft. Durch diese Leserasterverschiebung tritt an den Basen 404 bis 406 ein opal-Stopsignal auf. Damit ist das Produkt dieses Gens nur etwa ein Drittel so groß wie die funktionelle AlaDH der anderen Stämme.

Das entscheidende bei dieser dritten Abweichung in der Gensequenz ist die Tatsache, daß sie genau in den drei Stämmen auftritt, die keine AlaDH-Aktivität zeigen. *M.bovis* und *M.bovis BCG* sind die einzigen Stämme des *M.tuberculosis* Complex, die keine Aktivität zeigen. Alle anderen Stämme wurden als mäßig oder stark positiv klassifiziert. Die beobachtete Deletion ist also der Grund für das Fehlen einer funktionellen AlaDH. Da jedoch mit dem mAb HBT-10 auch nicht das verkürzte Protein nachgewiesen werden kann (das Epitop von HBT-10 liegt im Bereich vor der Leserasterverschiebung), ist davon auszugehen, daß das verkürzte Protein erst gar nicht bzw. nur in sehr kleinen, mit dem mAb HBT-10 nicht detektierbaren, Mengen, produziert wird.

### 4. AlaDH-Aktivität und AlaDH-Gen in Mykobakterien

**AlaDH-Aktivität in Mykobakterien.** Die gemessenen AlaDH-Aktivitäten lassen einige interessante Beobachtungen bezüglich der Lebensweise der Organismen mit positiver Aktivität zu.

Die Stämme mit starker Aktivität sind allesamt pathogen. Interessant ist hierbei, daß zwei der vier in diese Gruppe fallenden Stämme pathogen für Fische sind (Austin & Austin, 1987). Alle beide, *M.marinum* und *M.chelonae*, können jedoch auch Menschen infizieren (Wallace *et al*., 1983; Johnston & Izumi, 1987). Im Gegensatz zur Tuberkulose lösen sie jedoch meist krankhafte Infektionen der oberen Hautschichten aus, die meist relativ unproblematisch zu behandeln sind.

*M.chelonae* ist ein vergleichsweise schnell wachsendes, nichtchromogenes Bakterium. Infektionen beim Menschen treten oft als sekundäre Wundinfektionen nach Operationen auf (Cooper *et al*., 1989). *M.marinum* ist ein langsam wachsender Qrganismus, der bei Wachstum an Licht ein gelbes Pigment bildet. In mehr als 50 wechselwarmen Spezies (Reptilien, Amphibien, Fische) wurden Infektionen mit *M.marinum* nachgewiesen (Clark & Shepard, 1963). Beim Menschen manifestiert sich das Bakterium meist im Ellbogenoder Kniebereich.

Die beiden anderen Stämme mit stark-positiver AlaDH-Aktivität sind Vertreter des *M.tuberculosis* Complex. Es sind dies der Tuberkulose-Referenzstamm *M.tuberculosis* H37Rᵥ, sowie der Stamm *M.microti,* der als phylogenetisches Bindeglied zwischen *M.tuberculosis* und *M.bovis* betrachtet wird.

Bis auf *M.smegmatis* sind auch alle als mäßig-positiv klassifizierten Stämme pathogen. Der Großteil dieser Stämme umfasst klinische Isolate von *M.tuberculosis.* Pathogene Varianten von Tuberkulose-Stämmen scheinen also in der Regel AlaDH-Aktivität zu besitzen. Es wurden jedoch auch zwei Isolate gefunden, die keine AlaDH-Aktivität aufweisen. Der einzige nicht-pathogene Organismus mit AlaDH-Aktivität ist der schnell wachsende Stamm *M.smegmatis. M.smegmatis* weist sich jedoch durch eine ungewöhnlich starke NAD⁺-reduzierende Hintergrundaktivität aus, und ist. deshalb sehr leicht von allen anderen Stämmen mit AlaDH-Aktivität zu unterscheiden. Desweiteren wurde im Stamm *M.smegmatis* 1-2c, ein mykobakterieller Expressionsstamm, keine AlaDH-Aktivität gefunden.

Innerhalb der 44 getesteten Mykobakterien-Stämme, und dies ist bei weitem der Großteil aller bekannten Stämme, ist deshalb die Folgerung erlaubt:
⇒ Ein langsam wachsendes Mykobaktenum mit positiver AlaDH-Aktivität is virulent.

Der Umkehrschluß dieser Feststellung ist jedoch falsch. Unter den Stämmen ohne AlaDH-Aktivität sind etliche virulente. Trotzdem kommt man nicht umhin eine, wenn auch nicht feste, Tendenz festzustellen, nach der die AlaDH-Aktivität mit steigender Pathogenität eines Stammes zunimmt. Insbesondere durch die Aktivitäten der verschiedenen Stämme von *M.tuberculosis* wird diese

These unterstrichen. Die mit Abstand höchste Aktivität hat der Stamm H37Rᵥ, der als Referenzstamm für alle Tuberkulose-Laboratorien dient, und eine bekannt hohe Infektiösität besitzt. Ganz am Ende rangiert das avirulente Derivat von H37Rᵥ, der Stamm H37Rₐ. Zwischen diesen beiden Polen rangieren die klinischen Tuberkulose-Isolate, die mal etwas mehr und mal etwas weniger Aktivität zeigen.

**Das *AlaDH-Gen* in Mykobakterien.** Das Gen für die Alanin Dehydrogenase konnte in allen untersuchten Stämmen des *M.tuberculosis* Complex und im Stamm *M.marinum* identifiziert werden.

Der entscheidene Punkt beim Vergleich der Sequenzen innerhalb des *M.tuberculosis* Complex ist die Deletion der Base 272, die bei den untersuchten Stämmen von *M.bovis* und *M.bovis* BCG zu einer Leserasterverschiebung und letztendlich zu einem verkürzten, nicht-funktionellen Protein führt. Bei diesen Stämmen ließ sich auch in Zellextrakten keine AlaDH-Aktivität nachweisen. Diese Daten stimmen auch mit den Ergebnissen von Andersen *et al*. (1992) überein, die in Southern Blots zwar Signale mit diesen Stämmen erhielten, aber in Western Blots kein Protein nachweisen konnten.

Durch die Amplifikation und Sequenzierung des Gens konnte in dieser Arbeit die Ursache hierfür gefunden werden. Es muß jedoch auch in Betracht gezogen werden, daß noch weitere Änderungen in den regulatorischen Genabschnitten für das Fehlen des verkürzten Proteins verantwortlich sein können. Dies könnte eine Maßnahme der Zelle sein, keine Energie in ein nicht funktionsfähiges Protein zu investieren. Allgemein ist über regulatorische Gensequenzen bei Mykobakterien noch nicht viel bekannt (Dale & Patki, 1990; Gupta *et al.* 1993). Es scheint jedoch, daß, nach dem Prinzip von Enhancern, auch weiter weg gelegene Abschnitte die Genexpression nicht unerheblich beeinflußen können. Die für eine Einstellung der Produktion des Proteins nötigen Mutationen müssen also nicht zwangsläufig auf dem in dieser Arbeit sequenzierten Bereich liegen.

Das andere identifizierte *AlaDH-Gen*, jenes von *M.marinum,* ist auf DNA-Ebene deutlich unterschiedlich von den Genen des *M.tuberculosis* Complex. Immerhin vier von fünf Basen (80,4%) sind beim Vergleich dieser Sequenzen jedoch durchschnittlich noch identisch. Dieser Wert ist auf Proteinebene noch höher (85,3%. Identität, 92,0% Ähnlichkeit). Da AlaDH-Aktivität jedoch auch in einer Reihe weiterer Spezies gefunden wurde, ist davon auszugehen, daß sich die entsprechenden Gene, mangels Homologie zu den benutzten Primern, bei den benutzten Bedingungen nicht amplifizieren ließen. Eine eingehendere Studie betreffs dieses Punktes müsste auch diese Gene auffinden können. Ein Vergleich all dieser Sequenzen könnte weitere Rückschlüsse auf die Rolle des Enzyms zulassen.

Desweiteren ist denkbar, daß sich anhand eines solchen Sequenzvergleiches ein PCR-Verfahren entwickeln lassen müsste, mit dem man Mykobakterien, die ein *AlaDH*-Gen besitzen, voneinander unterscheiden kann. Und wie in dieser Arbeit gezeigt werden konnte, sind es eben die für den Menschen bedeutenden Stämme, die ein *AlaDH-Gen* besitzen. Besonders die Möglichkeit, mit einem solchen PCR-Assay den Erreger *M.tuberculosis* vom Impfstamm *M.bovis* BCG unterscheiden zu können, lassen ein solches Vorhaben interessant erscheinen.

**Ausblick.** Das in dieser Arbeit behandelte 40 kD-Antigen stellt in mehrererlei Hinsicht ein lohnenswertes Objekt für weitergehende Untersuchungen dar. Ein Punkt, der in dieser Arbeit nicht weiter berücksichtigt wurde, ist die mögliche Anwendung dieses Enzyms in der medizinischen Diagnostik. So wurden bereits für die Enzyme Dipeptidase (Ito et al., 1984), γ-Glutamyltransferase (Kondo *et al.*, 1992) und γ-Glutamyl Cyclotransferase (Takahashi et al., 1987) Assays beschrieben, die auf einer AlaDH basieren. Alle drei genannten Enzyme sind bei verschiedenen Krankheiten in veränderten Urin-, Serum- bzw. Blutkonzentrationen vorzufinden.

Das Hauptaugenmerk liegt jedoch auf dem Einsatz des 40 kD-Antigens bei der Tuberkulose. Ansatzpunkte sind hierbei an mehreren Stellen denkbar.

Allein bei der Diagnostik sind mehrere Möglichkeiten vorstellbar, wie das 40 kD-Antigen, bzw. das ihm zugrundeliegende Gen, genutzt werden könnte. Da das rekombinante Protein nun leicht aus dem überproduzierenden *E.coli* Stamm gewonnen werden kann, erscheint es lohnenswert, die Nützlichkeit dieses Proteins in der Serologie zu überprüfen. Zudem könnten sich diagnostische Verfahren entwickeln lassen, die auf dem direkten Nachweis von AlaDH-Aktivität oder, wie bereits erwähnt, auf der Amplifikation spezifischer Teile des Gens beruhen. Die Deletion der Base 272 in den Stämmen *M.bovis* und *M.bovis* BCG kann hierbei als Ansatzpunkt der Diskriminierung dieser beiden Stämme von *M.tuberculosis* dienen.

Ein PCR-Assay müsste sich auch für den Stamm *M.marinum* etablieren lassen, der sich ja auf Genebene nicht unerheblich vom *M.tuberculosis* Complex unterscheidet. Bislang wird zu diesem Zweck ein PCR-Assay, beruhend auf der Amplifikation eines Teils der für die 16S rRNA kodierenden Gensequenz, eingesetzt (Knibb et al., 1993). Dies ist, in Anbetracht der in den letzten Jahren steigenden Zahl von Infektionen mit *M.marinum* in Fischfarmen (Knibb et al., *1993*), von großer Bedeutung. Auch Infektionen beim Menschen werden in den letzten Jahren gehäuft vermeldet (Harris *et al.,* 1991; Kullavanijaya *et al.,* 1993; Slosarek *et al., 1994*).

Die Beobachtung, daß die Virulenz eines Stammes von *M.tuberculosis* sehr gut mit seiner AlaDH-Aktivität korreliert, wirft erneut die Frage auf, ob das Enzym einen Virulenzfaktor darstellt. Zur Beantwortung dieser Frage sind Ansätze denkbar, wie der *knock-out* des Gens in *M.tuberculosis* oder die Überexpression des Gens in einem Stamm mit niedriger Virulenz. In beiden Fällen kann die Virulenz im Tiermodell überprüft werden.

### 6. Anhänge

### Abkürzungsverzeichnis

- A: präexponentieller Faktor oder Stoßfaktor
- Aₓₓₓ: Absorption bei einer Wellenlänge von xxx nm
- AlaDH: L-Alanin Dehydrogenase (E.C. 1.4.1.1.)
- AMC: Academic Medical Centre, Amsterdam, Niederlande
- Ap: Ampicillin
- AP: Alkalische Phosphatase
- app.: apperent
- AS: Aminosäure
- ATCC: American Type Culture Collection, Rockville, USA
- ATP: Adenosin-Triphosphat
- BCG: Bacille Calmette Guérin
- BCIG: S-Bromo-4-Chloro-3-Indolyl-β-D-Galactopyranosid
- BCIP: 5-Bromo-4-Chloro-3-Indolylphosphat
- Boc: tert-Butoxycarbonyl
- bp: Basenpaar(e)

- cfu: colony forming units
- Cm: Chloramphenicol
- Conc: Konzentration

- DMEM: Dulbecco's Modified Eagle Medium
- DMF: Dimethylformamid
- DMSO: Dimethylsulfoxid
- DNA: Desoxyribonukleinsäure
- DTNB: Dithiobisnitrobenzoesäure
- DTT: Dithiothreitol

- Eₐ: Aktivierungsenergie
- EDTA: Ethylendiamintetraacetat
- Eth: Ethionamid

- F: Farad
- FBS: Fötales Rinderserum
- FCS: Fötales Kalbsserum
- Fmoc: 9-Flourenylmethoxycarbonyl
- FPLC: Fast Protein Liquid Chromatography
- frag.: Fragment

- g: Fallbeschleunigung
- GBF: Gesellschaft für biotechnologische Forschung mbH, Braunschweig, Deutschland
- GlcNAc: N-Acetylglucosamin
- Gm: Gentamicin
- GOGAT: Glutamin-Oxoglutarat-Aminotransferase
- GS: Glutamin-Synthetase
- GST: Glutathion-S-Transferase

- h: Stunde(n)
- HBSS: Hank's Balanced Salt Solution
- HIV: Human Immunodeficiency Virus
- HOBt: Hydroxybenzotriazol
- HRP: Horseradish Peroxidase
- Hsp: Hitzeschockproteine

- Ig: Immunglobulin
- IL: Interleukin
- INH: Isonicotinsäurehydrazid, Isoniazid
- IPTG: Isopropyl-β-D-thiogalactosid

- k: Umsatzrate eines Enzyms
- kb: Kilobasen
- KBL: Kilobasenleiter
- kD, kDa: Kilodalton
- KIT: Royal Tropical Institute, Amsterdam, Niederlande
- K_{M}: Michaelis-Konstante
- Km: Kanamycin

- MΦ: Makrophage(n)
- mAb: monoklonaler Antikörper
- MAIS: *M.avium - M.intracellulare - M.scrofulaceum* Complex
- MBP: maltose binding protein
- MCAC: Metallchelat-Affinitätschromatographie
- mesoDAP: meso Diaminopimelinsäure
- min: Minute(n)
- m.o.i.: multiplicity of infection
- MRC: Medical Research council, Tuberculosis and Related
Infections Unit, London, England
- MTT: Thiazolylbluetetrazoliumbromid
- MurNAc: N-Acetylmuraminsäure
- MurNGl: N-Glycolylmuraminsäure

- NAD⁺: Nicotinamidadenindinucleotid, oxidierte Form
- NADH: Nicotinamidadenindinucleotid, reduzierte Form
- NADP⁺: Nicotinamidadenindinucleotidphosphat, oxidierte Form
- NADPH: Nicotinamidadenindinucleotidphosphat, reduzierte Form
- n.b.: nicht bestimmt
- NBT: Nitrobluetetrazoliumchlorid
- Nr.: Nummer
- NTP: beliebiges Nucleosid in Form eines Triphosphats

- oD: oxidative Desaminierung
- ORF: open reading frame, offenes Leseraster
- OtBu: tert-Butylester
- PAGE: Polyacrylamid-Gelelektrophorese
- pac: protein antigen c, alte Bezeichnung für das 40 kD-Antigen
- PCR: polymerase chain reaction Polymerase-Kettenreaktion
- Pfp: Pentafluorphenyl
- PMA: Phorbolmyristatacetat
- Pmc: Pentamethylchroman
- PMS: Phenazinmethosulfat
- PNT: Pyridinnukleotid-Transhydrogenase
- PPD: Purified protein derivative
- PVDF: Polyvinylidendiflourid

- R: Rydberg-Konstante oder Resistenz (wenn Buchstabe hochgestellt)
- rA: reduktive Aminierung
- rec: rekombinant
- Rha: Rhamnose
- Rif: Rifampicin
- RIV: National Institute of Public Health and the Environment, Buthoven, Niederlande
- RNA: Ribonukleinsäure
- RNI: reaktive nitrogen intermediates, reaktive Stickstoff-Intermediate
- ROI: reaktive oxygen intermediates, reaktive Sauerstoff-Intermediate
- rpm: rounds per minute, Umdrehungen pro Minute
- rRNA: ribosomale Ribonukleinsäure
- RT: Raumtemperatur

- SDS: Natriumdodecylsulfat
- sec: Sekunde(n)
- Str: Streptomycin

- Tb: Tuberkulose
- TEMED: N.N.N',N'-Tetramethylethylendiamin
- TIR: Translations-Initiationsregion
- Tris: Tris(hydroxymethyl)-aminomethan
- Trt: Trityl
- ts: temperatur-sensitiv
- Tween: Polyoxyethylensorbitanmonolaurat

- U: Unit(s)
- ÜN: über Nacht
- unveröff.: unveröffentlicht

- vₘₐₓ: maximale Reaktionsgeschwindigkeit
- VMDC: Veterinary Microbiological Diagnöstic Centre, Utrecht, Niederlande
- Vol.: Volumen

- WHO: World Health Organization, Weltgesundheitsorganisation
- WKZ: Academisch Ziekenhuis, Utrecht, Niederlande

- z.A.: zur Analyse, von höchstem Reinheitsgrad

### Abkürzungen für Aminosäuren und Nukleotide

| Aminosäure | 3-Buchstaben-Code | 1-Buchstaben-Code |
|---|---|---|
| Alanin | Ala | A |
| Arginin | Arg | R |
| Asparagin | Asn | N |
| Aspartat | Asp | D |
| Cystein | Cys | C |
| Glutamin | Gin | Q |
| Glutamat | Glu | E |
| Glycin | Gly | G |
| Histidin | His | H |
| Isoleucin | Ile | I |
| Leucin | Leu | L |
| Lysin | Lys | K |
| Methionin | Met | M |
| Phenylalanin | Phe | F |
| Prolin | Pro | P |
| Serin | Ser | S |
| Threonin | Thr | T |
| Tryptophan | Trp | W |
| Tyrosin | Tyr | Y |
| Valin | Val | V |

| **Base** | **Nukleosid / Nukleotid** | **Abkürzung** |
|---|---|---|
| Adenin | Adenosin | A |
| Cytosin | Cytidin | C |
| Guanin | Guanosin | G |
| Uracil | Uridin | U |
| Thymin | Thymidin | T |

### 6. Literaturverzeichnis

Andersen A.B.; Andersen P.& Ljungqvist L. (1992). Structure and Function of a 40,000 - Molecular - Weight Protein Antigen from *Mycobacterium tuberculosis*. Infect. Immun. **60**, 2317-2323.
Austin B. & Austin D.A. (1987). Bacterial Fish Pathogens - Disease in Farmed and Wild Fish. Chapter 7 : Aerobic Gram-Positive Rods. Ellis Horwood Ltd., Chicester.
Bass Jr. J.B.; Farer L.S.; Hopewell P.C.; Jacobs R.F. & Snider Jr. D.E. (1990). Diagnostic Standards and Classification of Tuberculosis. Am.Rev.Resp.Dis. **142,** 725-735.
Cherayil B.J. & Young R.A. (1988). A 28-kDa Protein from *Mycobacterium leprae* is a Target of the Human Antibody Response in Lepromatous Leprosy. J.Immunot. **141,** 4370-4375.
Clark H.F.& Shepard C.C. (1963). Effect of Environmental Temperatures on Infection with *Mycobacterium marinum (balnei)* of Mice and a Number of Poikilothermic Species. J.Bacteriol. **86,** 1057-1069.
Cooper J.F.; Lichtenstein M.J.; Graham B.S. & Schaffner W. (1989). *Mycobaclerium chelonae* : A Cause of Nodular Skin Lesions with a Proclivity for Renal Transplant Recipients. Am.J.Med**. 86,** 173-177.
Dale J.W. & Patki A. (1990). Mycobacterial Gene Expression and Regulation. In : Molecular Biology of the Mycobacteria. (Ed. J.McFadden) Surrey University Press, London.
Delforge D.; Depiereux E.; De Bolle X.; Feytmans E. & Remacle J. (1993). Similarities Between Alanine Dehydrogenase and the N-Terminal Part of Pyridine Nucleotide Transhydrogenase and their Possibte Implication in the Virutence Mechanism of *Mycobacterium tuberculosis*. Biochem.Biophys.Res.Comm. **190,** 1073-1079.
van Eden W.; Thote J.E.; van der Zee R.; Noordzij A.; van Embden J.D.; Hensen E.J. & Cohen I.R. (1988). Cloning of the Mycobacterial Epitope Recognized by T Lymphocytes in Adjuvant Arthritis. Nature **331,** 171-173.
Garbe T.R.; Barathi J.; Barnini S.; Zhang Y.; Abou-Zeid C.; Tang D.; Mukherjee R. & Young D.B. (1994). Transformation of a Range of Mycobacterial Species using Hygromycin as Selectable Marker. Mol.Microbiol. **140,** 133-138.
Gottesman M.E.; Adhya S. & Das A. (1980). Transcription Antitermination by Bacteriophage Lambda *N* Gene Product. J.Mol.Biol. **140,** 57-75.
di Guan C.; Li P.; Riggs P.D. & Inouye H. (1987). Vectors that Faciliate the Expression and Purification of Foreign Peptides in *Escherichia coli* by Fusion to Maltose-Binding Protein. Gene **67,** 21-30.
Gupta S.K.;Bashyam M.D. & Tyagi A.K. (1993). Cloning and Assessment of Mycobacterial Promoters by Using a Plasmid Shuttle Vector. J.Bacteriol. **175**, 5186-5192.
Gupta S. & Tyagi A.K. (1993). Sequence of a Newly Identified *Mycobacterium tuberculosis* Gene Encoding a Protein with Sequence Homology to Virulence-Regulating Proteins. Gene **126**, 157-158.
Hanahan D. (1983). Studies on Transformation of *Escherichia coli* with Plasmids. J.Mol.Biol. **166,** 557-580.
Harris L.F.; Striplin W.H. & Burnside R.C. (1991). Aquatic Hazard *Mycobacterium marinum* Infection. Ala.Med. **61**, 8-10.
Huebner R.E.; Schein M.F. & Bass Jr. J.B. (1993). The Tuberculin Skin Test. Clin.Inf.Dis. **17,** 968-975.
Inagaki K.; Tanizawa K.; Badet B.; Walsh C.T.; Tanaka H. & Soda K. (1986). Thermostable Alanine Racemase from *Bacillus stearothermophilus* : Molecular Cloning of the Gene, Enzyme Purification and Characterization. Biochemistry **25,** 3268-3274.
Ito Y.; Watanabe Y.; Hirano K.; Sugiura M.; Sawaki S. & Ogiso T. (1984). A Fluorometric Method for Dipeptidase Activity Measurementn in Urine, Using L-Alanyl-L-Alanine as Substrate. J.Biochem. **96,** 1-8.
Johnston J.M & Izumi A.K. (1987). Cutaneous *Mycobacterium marinum* Infection ("Swimming Pool Granuloma"). Clin.Dermatol. **5**, 68-75.
Khanolkar-Young S.; Kolk A.H.J.; Andersen A.B.; Bennedsen J.; Brennan P.J.; Rivoire B.; Kuijper S,; McAdam K.P.W.J.; Abe C.; Batra H.V.; Chaparas S.D.; Damiani G.; Singh M. & Engers H.D. (1992). Results of the Third Immunology of Leprosy / Immunology of Tuberculosis Antimycobacterial Monoclonal Antibody Workshop. Infect.Immun. **60**, 3925-7.
Knibb W.; Colorni A.; Ankaoua M.; Lindell D.; Diamant A. & Gordin H. (1993). Detection and Identification of a Pathogenic Marine Mycobacterium from the European Seabass *Dicentrarchus labrax* Using Polymerase Chain Reaction and Direct Sequencing of 16S rDNA Sequences. Mol.Mar.Biol.Biotechnol. **2**, 225-232.
Kondo H.; Hashimoto M.; Nagata K.; Tomita K & Tsubota H. (1992). Assay of γ-Glutamyltransferase with Amino Acid Dehydrogenases from *Bacillus sfearothermophilus* as Auxilary Enzymes. Clin.Chim.Acta **207,** 1-9.
Kullavanijaya P.; Sirimachan S. & Bhuddhavudhikrai P. (1993). *Mycobacterium marinum* Cutaneous Infections Acquired from Occupations and Hobbies. Int.J.Dermatol. **32,** 504-507.
McCarthy J.E.G.; Sebald W.; Gross G. & Lammers R. (1986). Enhancement of Translation Efficiency by the *Escherichia coli alpE* Translation Initiation Region : Its Fusion with Two Human Genes. Gene **41,** 201-206.
Mehra V; Sweetser D. & Young R.A. (1986). Efficient Mapping of Protein Antigenic Determinants. Proc.Natl.Acad.Sci. **83**,7013-7017.
Mehra V.; Bloom B.R.; Bajardi A.C.; Grisso C.L.; Sieling P.A.; Alland D.; Convit J.; Fan X.D.; Hunter S.W. & Brennan P.J.; Rea T.H. & Modlin R.L. (1992). A Major T Cell Antigen of *Mycobacterium leprae* Is a 10-kD Heat-Shock Cognate Protein. J.Exp.Med. **175,** 275-284.
Ralph P. & Nakoinz I. (1975). Phagocylosis and Cytolysis by a Macrophage Tumor and its Cloned Cell Line. Nature **257**, 393-394.
Ralph P. & Nakoinz I. (1976). Lysozyme Synthesis by a Established Human and Murine Histiocytic Lymphoma Cell Line. J.Exp.Med. **143.**1528-1533.
Sambrook J.; Fritsch E.F. & Maniatis T. (1989). Molecular Cloning - A Laboratory Manual, Second Edition. Cold Spring Harbor Laboratory Press, Cold Spring Harbor.
Schauder B.; Blöcker H.; Frank R. & McCarthy J.E.G. (1987). Inducible Expression Vectors Incorporating the *Escherichia coli alpE* Translation Initiation Region. Gene **52,** 279-283.
Singh M.; Andersen A.B.; McCarthy J.E.G.; Rohde M.; Schütte H.; Sanders E. & Timmis K.N. (1992). The *Mycobaclerium tuberculosis* 38-kDa antigen : Overproduction in *Escherichia coli,* Purification and Characterization. Gene **117**, 53-60.
Slosarek M.; Kubin M. & Pokorny J. (1994). Water as a Possible Factor of Transmission in Mycobacterial Infections. Cent.Eur.J.Public Health **2**, 103-105.
Snyderman R.; Pike M.C.; Fischer D.G. & Koren H.S. (1977). Biologic and Biochemicat Activities of Continuous Macrophage Cell Lines P338D1 and J774.I. J.Immunol. **119,** 2060-2066.
Studier F.W. (1975). Genetic Mapping of a Mutation that Causes Ribonuclease III Deficiency in *Escherichia coli*. J.Bacteriol. **124,** 307-316.
Takahashi T.; Kondo T.; Ohno H.; Minato S.; Ohshima T.; Mikuni S.; Soda K. & Taniguchi N. (1987). A Spectrophotometric Method for the Determination of γ-Glutamyl Cyclotransferase with Alanine Dehydrogenase in the Presence of Anthglutin. Biochem.Med.Metabol.Biol. **38,** 311-316.
Thole J.E.; Keulen W.J.; de Bruyn J.; Kolk A.H.; Groothuis D:G.; Berwald L.G.; Tiesjema R.H. & van Embden J.D. (1987). Characterization, Sequence Determination, and Immunogenicity of a 64-Kilodalton Protein of *Mycobacterium bovis* BCG Expressed in *Escherichia coli* K-12. Infect.Immun. **55,** 1466-1475.
Thole J.E.; Stabel L.F.; Suykerbuyk M.E.; de Wit M.Y.; Klatser P.R.; Kolk A.H. & Hartskeerl R. (1990). A Major Immunogenic 36,000-Molecular-Weight Antigen from *Mycobacterium leprae* Contains an Immunoreactive Region of Proline-Rich Repests. Infect.Immun. **58,** 80-87.
Verbon A.; Hartskeerl R.A.; Schuitema A; Kolk A.H.J.; Young D.B. & Lathigra R. (1992). The 14,000-Molecular-Weight Antigen of *Mycobacterium tuberculosis* is Related to the *Alpha-Crystallin* Family of Low-Molecular-Weight Heat Shock Proteins. J.Bacteriol. **174,** 1352-1359.
Vordermeier H.M.; Harris D.P.; Lathigra R.; Roman E.; Moreno C. & Ivanyi J. (1993). Recognition of Peptide Epitopes of the 16,000 MW Antigen of *Mycobacterium tuberculosis* by Murine T Cells. immunology **80,** 6-12.
Wallace Jr. R.J.; Swenson J.M.; Silcox V.A.; Good R.C.; Tschen J.A. & Stone M.S. (1983). Spectrum of Disease Due to Rapidly Growing Mycobacteria. Rev.Infect.Dis. **5**, 657-679.
Zhang Y.; Lathigra R.; Garbe T.; Catty D. & Young D. (1991). Genetic Analysis of Superoxide Dismutase, the 23 Kilodalton Antigen of *Mycobacterium tuberculosis.* Mol.Microbiol. **5**, 381-391.

## Patentansprüche

1. Enzymatischer Test-Kit zur Diagnose von Tuberkulose und anderen mycobakteriellen Infektionen in Menschen und Tieren durch Bestimmung der Aktivität von Alanindehydrogenase (E.C. 1.4.1.1) umfassend L-Alanin, Nicotinamidadenin-dinucleotid (oxidierte Form; NAD⁺), Phenazinmethosulfat (PMS) und Nitrobluetetrazoliumchlorid (NBT).

2. Verfahren zur Diagnose von Tuberkulose und anderen mycobakteriellen Infektionen von Menschen und Tieren, **dadurch gekennzeichnet, dass** man mit einem enzymatischen Test-Kit gemäß Anspruch 1 die Aktivität von Alanindehydrogenase (E.C. 1.4.1.1.) misst.

3. Verfahren nach Anspruch 2, **dadurch gekennzeichnet, dass** man
(i) mögliche Tuberkulose-Erreger, wie *M. tuberculosis* isoliert,
(ii) einen Zellrohextrakt herstellt,
(iii) den Extrakt in Lösung inkubiert und
(iv) die Absorption misst.

4. Verfahren nach Anspruch 2 und/oder 3, **dadurch gekennzeichnet, dass** man klinische Proben, wie Körperflüssigkeiten, direkt einer Tuberkulosediagnose unterwirft und die Alanindehydrogenase-Aktivität misst.

5. Verfahren nach Anspruch 2, **dadurch gekennzeichnet, dass** man Zellen, Stämme und/oder Spezies von Krankheitserregern (Mycobakterien) von nicht-virulenten Zellen und Stämmen differenziert.

6. Verfahren nach Anspruch 5, **dadurch gekennzeichnet, dass** man Zellen, Stämme und/oder Spezies von Krankheitserregern des M. tuberculosis-Komplexes identifiziert und differenziert.

7. Verfahren nach einem der vorhergehenden Ansprüche, **dadurch gekennzeichnet, dass** man das Verfahren in Gegenwart von Tuberkulose und anderem mycobakterielle Infektionen von Menschen und Tieren inhibierenden Substanzen durchführt und diese inhibierenden Substanzen gegebenenfalls gewinnt.

8. Verfahren nach einem der vorhergehenden Ansprüche, **dadurch gekennzeichnet, dass** man es
(i) zur Epidemiebekämpfung und/oder
(ii) nach Impfungen (vaccination follow-up) bei Menschen und Tieren durchführt.

9. DNA-Sequenz des Alanindehydrogenase-Gens von *M. tuberculosis*, ausgewählt aus der folgenden Gruppe: 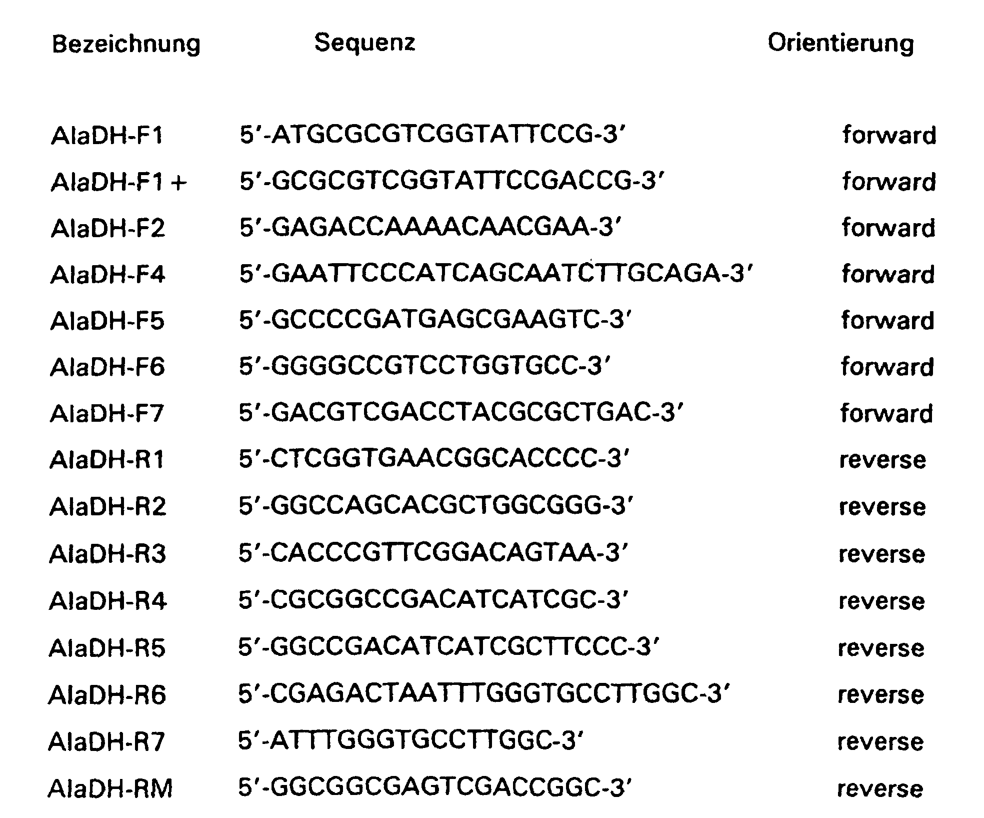 sowie Teilsequenzen davon zur Diagnose von Tuberkulose und anderen mykobakteriellen Infektionen bei Menschen und Tieren.

10. Verwendung einer DNA-Sequenz gemäß Anspruch 9 zur Diagnose von Tuberkulose und anderen mycobakteriellen Infektionen bei Menschen und Tieren.

11. Verfahren nach Anspruch 10, **dadurch gekennzeichnet, dass** man eine DNA-Sequenz gemäß Anspruch 9
(i) zum Hybridisieren,
(ii) zur Bestätigung von Kulturen (culture confirmation) isolierter Stämme und/oder
(iii) für chromosomales Fingerprinting einsetzt und Zeiten, Stämme und/oder Arten von Mycobakterien ermittelt und differenziert und/oder für die Diagnose von mycobakteriellen Infektionen einsetzt.

12. Verfahren nach Anspruch 10 oder 11, **dadurch gekennzeichnet, dass** man Zellen, Stämme und/oder Spezies von virulenten Mycobakterien von nicht-virulenten Zellen, Stämme und/oder Spezies differenziert.

13. Verfahren nach Anspruch 10, **dadurch gekennzeichnet, dass** man Zellen, Stämme und/oder Spezies des M. tuberculosis-Komplexes und anderer Mycobakterien
(i) isoliert,
(ii) rohe oder gereinigte genomische DNA oder RNA gewinnt,
(iii) ein Fragment identifiziert, das mit der Sequenz des Alanindehydrogenase-Gens von M. tuberculosis (Fig. 2.3) identisch oder praktisch identisch ist, vorzugsweise durch Amplifizieren unter Verwendung einer DNA-Sequenz gemäß Anspruch 9 als Primersequenz, wonach man mit einem Restriktionsenzym verdaut, insbesondere Bg1II, und eine Gelelektrophorese der verdauten amplifizierten DNA durchführt und/oder die DNA-Sequenz der amplifizierten DNA bestimmt.

14. Verfahren nach Anspruch 2 und/oder 10, **dadurch gekennzeichnet, dass** man eine klinische Probe unmittelbar einsetzt und auf Tuberkulose bei Menschen und Tieren diagnostiziert.

15. Verfahren nach Anspruch 2 und/oder 10, **dadurch gekennzeichnet, dass** man das Verfahren in Gegenwart von Tuberkulose oder mycobakterielle Infektionen von Menschen und Tieren inhibierenden Substanzen durchführt und ermittelte inhibierende Substanzen gewinnt oder herstellt.

16. Verfahren nach Anspruch 10, **dadurch gekennzeichnet, dass** man es
(i) bei antimycobakterieller Chemotherapie,
(ii) bei der Epidemiebekämpfung und/oder
(iii) nach Impfungen (vaccination follow-up) bei Menschen und Tieren anwendet.

## Claims

1. Enzymatic assay kit for the diagnosis of tuberculosis and other mycobacterial infections in humans and animals by determining alanine dehydrogenase (E.C. 1.4.1.1) activity, comprising L-alanine, nicotinamide adenine dinucleotide (oxidized form; NAD⁺), phenazine methosulphate (PMS) and nitroblue tetrazolium chloride (NBT).

2. Process for the diagnosis of tuberculosis and other mycobacterial infections of humans and animals, **characterized in that** alanine dehydrogenase (E.C. 1.4.1.1) activity is measured using an enzymatic assay kit according to Claim 1.

3. Process according to Claim 2, **characterized in that**
(i) possible tuberculosis pathogens such as *M. tuberculosis* are isolated,
(ii) a crude cell extract is prepared,
(iii) the extract is incubated in solution and
(iv) absorption is measured.

4. Process according to Claims 2 and/or 3, **characterized in that** clinical samples such as body fluids are subjected directly to tuberculosis diagnosis and alanine dehydrogenase activity is measured.

5. Process according to Claim 2, **characterized in that** cells, strains and/or species of pathogens (mycobacteria) are discriminated from non-virulent cells and strains.

6. Process according to Claim 5, **characterized in that** cells, strains and/or species of pathogens of the M. tuberculosis complex are identified and discriminated.

7. Process according to any of the preceding claims, **characterized in that** the process is carried out in the presence of substances inhibiting tuberculosis and other mycobacterial infections of humans and animals and, where appropriate, the said inhibiting substances are obtained.

8. Process according to any of the preceding claims, **characterized in that** it is carried out
(i) for controlling epidemics and/or
(ii) as vaccination follow-up in humans and animals.

9. DNA sequence of the *M. tuberculosis* alanine dehydrogenase gene, selected from the following group: 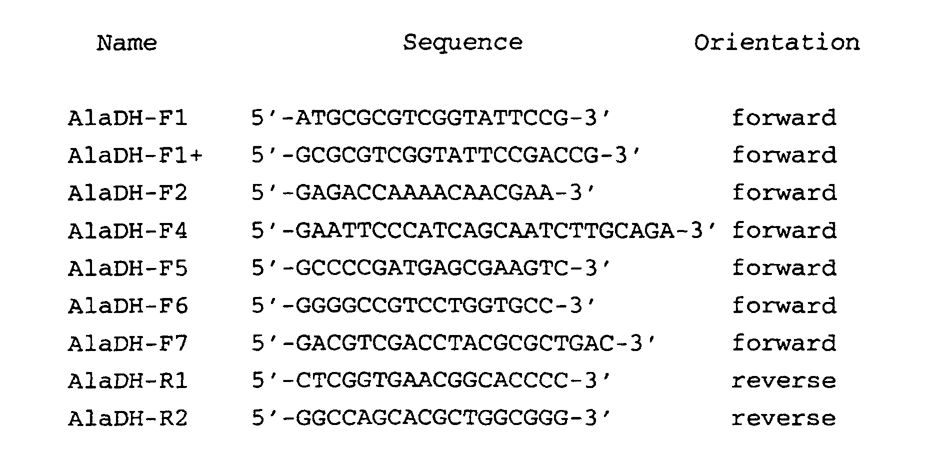 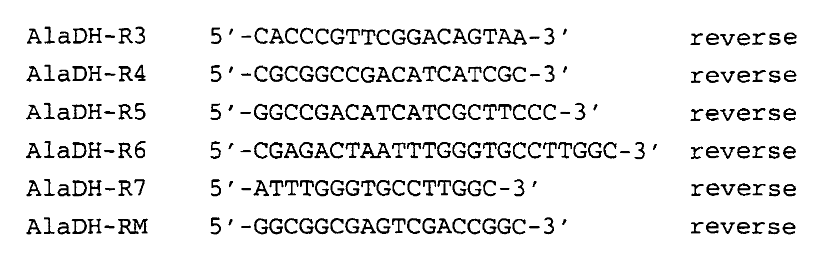 and also partial sequences thereof for the diagnosis of tuberculosis and other mycobacterial infections in humans and animals.

10. Use of a DNA sequence according to Claim 9 for the diagnosis of tuberculosis and other mycobacterial infections in humans and animals.

11. Use according to Claim 10, **characterized in that** a DNA sequence according to Claim 9 is employed
(i) for hybridizing,
(ii) for confirming cultures of isolated strains and/or
(iii) for chromosomal fingerprinting, and cells, strains and/or mycobacterial species are determined and discriminated and/or employed in the diagnosis of mycobacterial infections.

12. Process according to Claim 10 or 11, **characterized in that** cells, strains and/or species of virulent mycobacteria are discriminated from non-virulent cells, strains and/or species.

13. Process according to Claim 10, **characterized in that** cells, strains and/or species of the M. tuberculosis complex and of other mycobacteria are
(i) isolated,
(ii) crude or purified genomic DNA or RNA is obtained,
(iii) a fragment which is identical or virtually identical to the sequence of the *M.* tuberculosis alanine dehydrogenase gene (Fig. 2.3) is identified, preferably by amplification using a DNA sequence according to Claim 9 as a primer sequence, whereafter a digestion with a restriction enzyme, in particular BglII, and a gel electrophoresis of the digested amplified DNA are carried out and/or the DNA sequence of the amplified DNA is determined.

14. Process according to Claims 2 and/or 10, **characterized in that** a clinical sample is used directly and diagnosed for tuberculosis in humans and animals.

15. Process according to Claims 2 and/or 10, **characterized in that** the process is carried out in the presence of substances inhibiting tuberculosis or mycobacterial infections of humans and animals and inhibiting substances which have been determined are obtained or prepared.

16. Process according to Claim 10, **characterized in that** it is applied
(i) in anti-mycobacterial chemotherapy,
(ii) in controlling epidemics and/or
(iii) as vaccination follow-up in humans and animals.

## Revendications

1. Kit de test enzymatique pour le diagnostic de la tuberculose et d'autres infections mycobactériennes chez les humains et les animaux par la détermination de l'activité de la déhydrogénase de l'alanine (E.C. 1.4.1.1) comprenant la L-alanine, la dinucléotide de la nicotinamidadénine (forme oxydée ; NAD⁺), le méthosulfate de phénazine (PMS) et le chlorure de nitrobluetétrazolium (NBT).

2. Procédé pour le diagnostic de la tuberculose et autres infections mycobactériennes chez les humains et les animaux, **caractérisé en ce qu'**on mesure l'activité de la déhydrogénase de l'alanine (E.C. 1.4.1.1) au moyen d'un kit de test enzymatique selon la revendication 1.

3. Procédé selon la revendication 2, **caractérisé en ce qu'**on
(i) isole un éventuel microbe de la tuberculose tel que le *M. tuberculosis,*
(ii) prépare un extrait cellulaire brut,
(iii) incube l'extrait dans une solution et
(iv) mesure l'absorption.

4. Procédé selon la revendication 2 et/ou 3, **caractérisé en ce qu'**on soumet directement à un diagnostic de la tuberculose des échantillons cliniques tels que des liquides corporels et on mesure l'activité de la déhydrogénase d'alanine.

5. Procédé selon la revendication 2, **caractérisé en ce qu'**on différencie des cellules, des souches et/ou des espèces de microbes engendrant des maladies (des mycobactéries) par rapport à des cellules et des souches non virulentes.

6. Procédé selon la revendication 5, **caractérisé en ce qu'**on identifie et différencie des cellules, des souches et/ou des espèces de microbes parmi les complexes du M. tuberculosis.

7. Procédé selon l'une des revendications précédentes, **caractérisé en ce qu'**on conduit le procédé en présence de substances susceptibles d'inhiber la tuberculose et autres infections mycobactériennes chez les humains et les animaux et **en ce qu'**on recueille éventuellement ces substances susceptibles d'inhiber.

8. Procédé selon l'une des revendications précédentes, **caractérisé en ce qu'**on le conduit
(i) pour combattre une épidémie et/ou
(ii) après vaccination chez les humains et les animaux.

9. Séquence d'ADN du gène de la déhydrogénase de l'alanine du *M tuberculosis* choisie dans les groupes suivants : 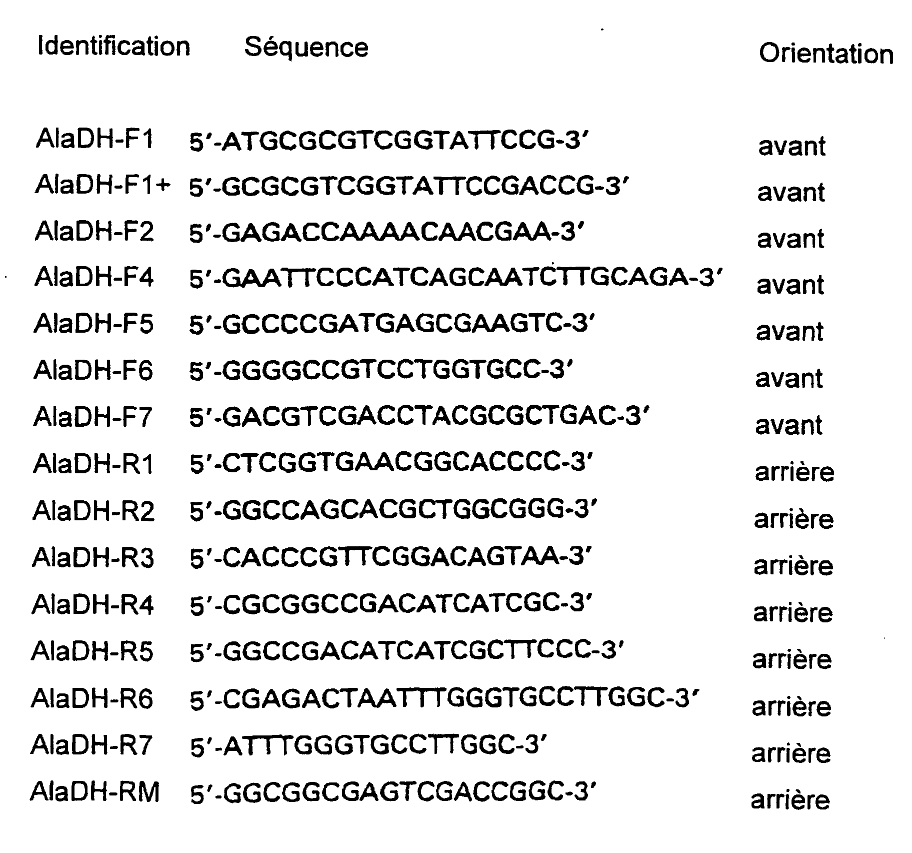 ainsi que des séquences partielles de celles-ci pour le diagnostic de la tuberculose et autres infections mycobactériennes chez les humains et les animaux.

10. Utilisation d'une séquence ADN selon la revendication 9 pour le diagnostic de la tuberculose et autres infections mycobactériennes chez les humains et les animaux.

11. Procédé selon la revendication 10, **caractérisé en ce qu'**on utilise une séquence ADN selon la revendication 9
(i) pour l'hybridation,
(ii) pour la détermination de souches isolées dans des cultures,
(iii) pour effectuer des empreintes chromosomiques pour déterminer et différencier des cellules, des souches et/ou d'autres espèces de mycobactéries et/ou pour le diagnostic d'infections mycobactériennes.

12. Procédé selon la revendication 10 ou 11, **caractérisé en ce qu'**on différencie des cellules, des souches et/ou autres espèces de mycobactéries virulentes à l'égard de cellules, souches et/ou espèces non virulentes.

13. Procédé selon la revendication 10, **caractérisé en ce qu'**on
(i) isole,
(ii) recueille de l'ADN ou de l'ARN génomique brut ou purifié,
(iii) identifie un fragment qui est identique ou pratiquement identique à la séquence du gène de la déhydrogénase de l'alanine du M. tuberculosis (fig. 2.3), de préférence par amplification en utilisant une séquence ADN selon la revendication 9 en tant que séquence primaire, après quoi on effectue une digestion avec une enzyme de restriction, en particulier Bg1II et une électrophorèse sous gel de l'ADN amplifié et digéré et/ou on détermine la séquence ADN de l'ADN amplifié.

14. Procédé selon la revendication 2 et/ou 10, **caractérisé en ce qu'**on utilise directement un échantillon clinique et on diagnostique la tuberculose chez les humains et les animaux.

15. Procédé selon la revendication 2 et/ou 10, **caractérisé en ce qu'**on conduit le procédé en présence de substances susceptibles d'inhiber la tuberculose ou autres infections mycobactériennes chez les humains et les animaux et on recueille ou prépare les substances susceptibles d'inhiber.

16. Procédé selon la revendication 10, **caractérisé en ce qu'**on l'utilise
(i) en chimiothérapie antimycobactérienne,
(ii) pour lutter contre les épidémies,
(iii) après vaccination chez les humains et les animaux.
